# NEW EUROPEAN PATENT SPECIFICATION

(11) **EP 2 651 400 B2**
(45) Date of publication and mention of the opposition decision: **18.01.2023**
(45) Mention of the grant of the patent: 07.08.2019
(21) Application number: 11831805.4
(22) Date of filing: 15.12.2011
(51) Int. Cl.: A61K 9/22, A61K 9/24, A61K 31/4035

(54) **CONTROLLED RELEASE ORAL DOSAGE FORMS OF POORLY SOLUBLE DRUGS AND USES THEREOF**
ORALE RETARD-DARREICHUNGSFORMEN VON SCHWER LÖSLICHEN ARZNEIMITTELN UND IHRE VERWENDUNG
FORMES POSOLOGIQUES POUR DES MÉDICAMENTS PEU SOLUBLES ADMINISTRÉS PAR VOIE ORALE À LIBÉRATION CONTRÔLÉE ET LEURS UTILISATIONS

(30) Priority: 16.12.2010 US 424003 P
(43) Date of publication of application: 23.10.2013
(73) Proprietor: Amgen (Europe) GmbH, 6343 Risch-Rotkreuz (CH)
(72) Inventor: CHEN, Ming, J., West Windsor, NJ 08550 (US); HUI, Ho-Wah, Basking Ridge, NJ 07920 (US); SHEN, Xiaole, Livingston, NJ 07039 (US)
(74) Representative: Carpmaels & Ransford LLP
(86) International application number: PCT/US2011/065151
(87) International publication number: WO 2012/083017

(56) References cited:
- WO-A1-2009/120167
- US-A- 4 814 176
- US-A1- 2004 254 214
- US-B1- 6 251 430
- LATCHMAN, LEIBERMAN et al.: "Chapter 14: Sustained Release Dosage Forms", The Theory and Practice of Industrial Pharmacy, pages 430-459,
- Gul Majid Khan: "Controlled Release Oral Dosage Forms: Some Recent Advances in Matrix Type Drug Delivery Systems", The Sciences, vol. 1, no. 5, 1 October 2001 (2001-10-01) , pages 350-354,
- de la Torre, P.M. et al: "Interpolymer complexes of poly(acrylic acid) and chitosan: influence of the ionic hydrogel-forming medium", Biomaterials, vol. 24, no. 8, 1 April 2003 (2003-04-01), pages 1459-1468,
- Zhilei Lu, Et Al: "Matrix Polymeric Excipients: Comparing a Novel Interpolyelectrolyte Complex with Hydroxypropylmethylcellulose", Drug Delivery, vol. 15, no. 2, 1 January 2008 (2008-01-01), pages 87-96,
- Moustafine, R.I. et al: "Characteristics of interpolyelectrolyte complexes of Eudragit E100 with Eudragit L100", Journal of Controlled Release, vol. 103, no. 1, 2 March 2005 (2005-03-02) , pages 191-198,

## Description

### 1. FIELD OF INVENTION

Provided herein are controlled release oral dosage forms of poorly soluble drugs, methods of making the dosage forms, and methods of their use for the treatment of various diseases and/or disorders. References in the description to treatments or to methods of treatment refer to the dosage forms of the present invention for use in a method of treatment.

### 2. BACKGROUND OF THE INVENTION

One goal in developing a drug is to provide dosage forms which make it possible to maintain a certain amount or concentration of drug in a subject's body that will remain constant for several hours. Often this may not be achieved by traditional rapidly disintegrating tablets, as these tablets release the active ingredient contained therein all at once. For this reason, dosage forms have been developed which are capable of continuously releasing the drug contained therein in a controlled manner and over a prolonged period of time. Oral controlled drug delivery is typically by solid dosage forms including tablets, capsules, microspheres, granules and suspensions.

Gastroretentive systems, drug delivery systems having a prolonged retention time in the stomach, represent a promising approach to controlled release oral delivery of drugs. Many such systems have been developed. For example, U.S. Patent Nos. 6,635,280 and 6,723,340 describe compositions for gastric retentive tablets which, upon oral administration, swell to a size such that the tablet cannot move out of the stomach easily. The drug is incorporated into a polymer matrix as the tablet swells and is released from the matrix into the gastric fluid by solution diffusion. *See* U.S. Patent No. 6,635,280. Thus, the tablet acts as a controlled released gastroretentive system. Other similar gastroretentive systems are described in the art. *See, e.g.,* European Patent No. EP 941071 B1. WO 2009/120167 discloses tablets with apremilast, microcrystalline cellulose, Pluronic, croscarmellose sodium and magnesium stearate.

A variety of polymeric excipients designed to expand or swell in the stomach have been used for the preparation of gastroretentive systems. *See e.g.,* U.S. Patent Nos. 6,210,710; 6,217,903; 5,945,125; 5,451,409; 4,915,952; U.S. Patent Publication Nos. 2003/0104053; 2003/0104062; and 2010/0129445. Such systems have been employed for the controlled release of poorly soluble drugs in particular. *See, e.g.,* U.S. Patent No. 6,635,280 and International Publication No. WO 97/22335. However, there exists a need for alternative controlled release dosage forms for drugs having poor aqueous solubility. Provided herein are controlled release dosage forms addressing this need.

### 3. SUMMARY OF THE INVENTION

Provided herein are controlled release oral dosage forms of the poorly soluble drug of formula (I), as defined in the claims, and, methods of making the solid forms, and methods of their use for the treatment of various diseases and/or disorders.

The controlled release oral dosage forms provided herein comprise polymeric excipients which expand and/or become charged in the gastric fluid in acidic pH and control the release of the poorly soluble drug in the system.

Without being bound to a particular theory, the controlled release oral dosage forms provided herein are believed to enhance the bioavailability of a poorly soluble drug by increasing the time of release of the drug in the gastrointestinal tract. In some embodiments, the extended time of release of the poorly soluble drug occurs mainly in the stomach.

Disclosed herein are also controlled release oral dosage forms that comprise positively charged polymers, negatively charged polymers and swelling excipients, which when combined with a poorly soluble drug in particular weight ratios of ingredients provide controlled release of the poorly soluble drug. Without being bound to a particular theory, controlled release of the poorly soluble drug is achieved by action of the swelling excipients and the interaction of the polymers containing negative charges and positive charges in acidic pH of the stomach or upper gastrointestinal tract.

In one embodiment, the controlled release oral dosage form comprises the following: (i) the poorly soluble drug of formula (I); (ii) a swelling excipient; (iii) a cationic polymer in acidic pH; and (iv) an anionic polymer in acidic pH as defined in the claims. In some embodiments, the controlled release oral dosage form further comprises a water absorbing agent. In some embodiments, the controlled release oral dosage form further comprises one or more additional pharmaceutically acceptable excipients.

The poorly soluble drug is (S)-*N*-{2-[1-(3-ethoxy-4-methoxy-phenyl)-2-methanesulfonylethyl]-1,3-dioxo-2,3-dihydro-1*H*-isoindol-4-yl}acetamide (Compound A).

In other described aspects, a poorly soluble drug is cyclopropanecarboxylic acid {2-[(1*S*)-1-(3-ethoxy-4-methoxy-phenyl)-2-methanesulfonyl-ethyl]-3-oxo-2,3-dihydro-1*H*-isoindol-4-yl}-amide (Compound B).

(*S*)-*N*-{2-[1-(3-ethoxy-4-methoxy-phenyl)-2-methanesulfonylethyl]-1,3-dioxo-2,3-dihydro-1*H*-isoindol-4-yl} acetamide (Compound A) has the following structure:

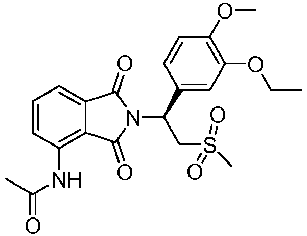

As disclosed herein, cyclopropanecarboxylic acid {2-[(*1S*)-1-(3-ethoxy-4-methoxy-phenyl)-2-methanesulfonyl-ethyl]-3-oxo-2,3-dihydro-1*H*-isoindol-4-yl}-amide (Compound B) has the following structure:

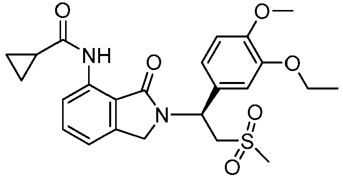

Provided herein are methods of treating, preventing or managing disorders ameliorated by the reduction of levels of TNF-α in a patient which comprises administering to a patient in need of such treatment, prevention or management a therapeutically or prophylactically effective amount of a compound provided herein, or a pharmaceutically acceptable polymorph, solvate, or hydrate thereof.

### 3.1. BRIEF DESCRIPTION OF THE FIGURES

FIG. 1 shows the drug release profile of Compound A in Formulations 1 to 3 over 24 hours.
FIG. 2 shows the drug release profile of Compound A in Formulations 4 to 7 over 24 hours.
FIG. 3 shows the drug release profile of Compound A in Formulations 8 to 11 over 24 hours.
FIG. 4 shows the drug release profile of Compound A in Formulations 12 to 15 over 24 hours.
FIG. 5 shows the drug release profile of Compound A in Formulations 16 to 25 over 24 hours.
FIG. 6 shows shows the drug release profile of Compound A in Formulations 28 to 33 over 24 hours.

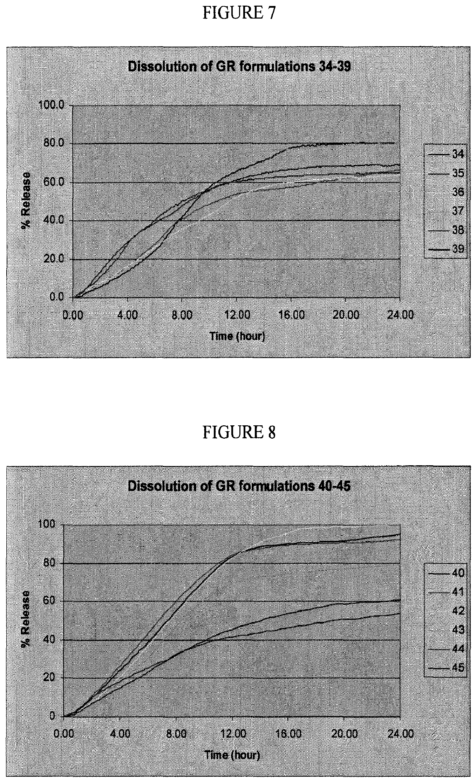

FIG. 7 shows shows the drug release profile of Compound A in Formulations 34 to 39 over 24 hours.
FIG. 8 shows shows the drug release profile of Compound A in Formulations 40 to 45 over 24 hours.

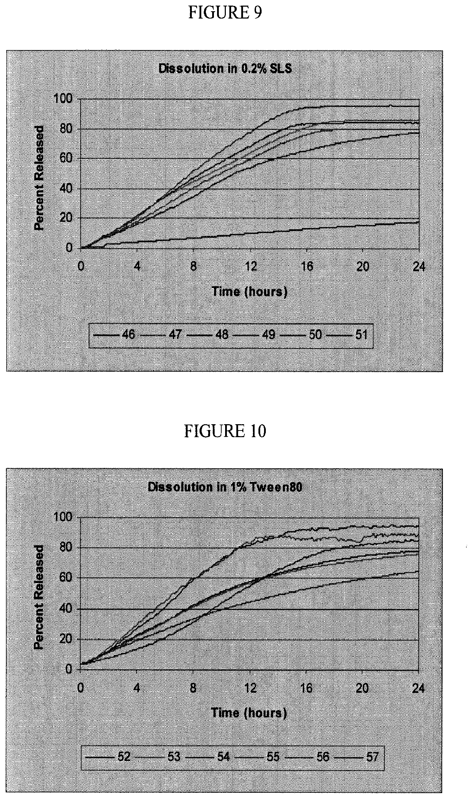

FIG. 9 shows shows the drug release profile of Compound A in Formulations 46 to 51 over 24 hours.
FIG. 10 shows shows the drug release profile of Compound A in Formulations 52 to 57 over 24 hours.

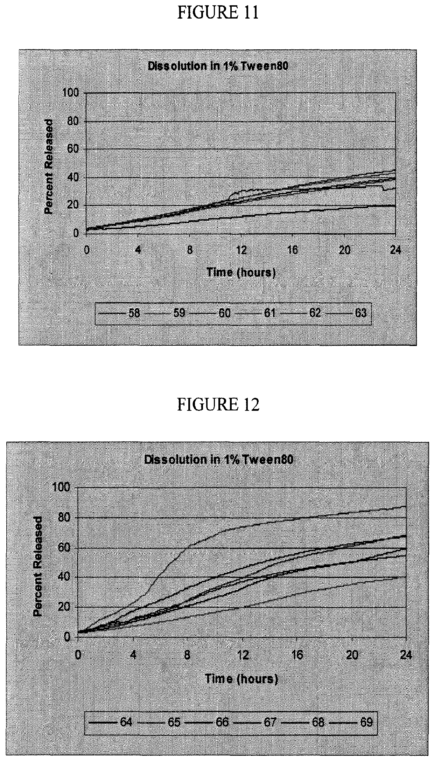

FIG. 11 shows shows the drug release profile of Compound A in Formulations 58 to 63 over 24 hours.
FIG. 12 shows shows the drug release profile of Compound A in Formulations 64 to 69 over 24 hours.

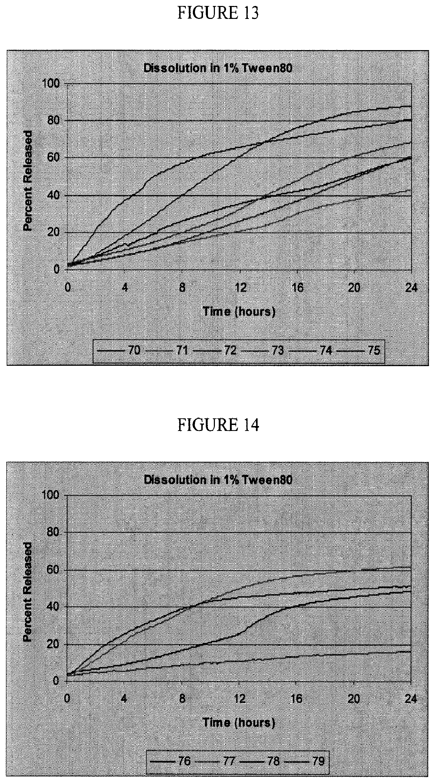

FIG. 13 shows shows the drug release profile of Compound A in Formulations 70 to 75 over 24 hours.
FIG. 14 shows shows the drug release profile of Compound A in Formulations 76 to 79 over 24 hours.

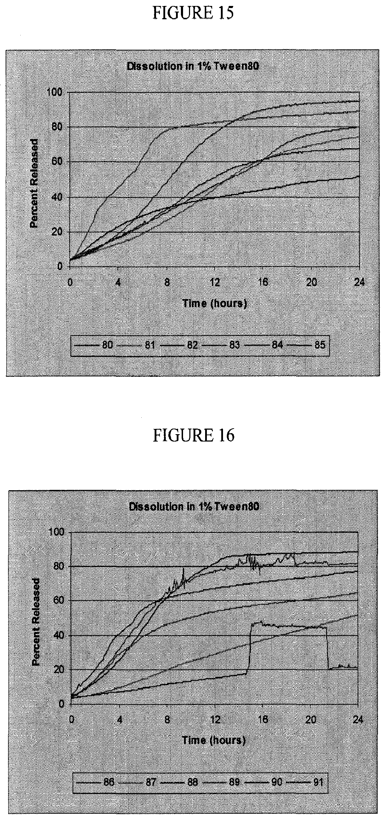

FIG. 15 shows shows the drug release profile of Compound A in Formulations 80 to 85 over 24 hours.
FIG. 16 shows shows the drug release profile of Compound A in Formulations 86 to 91 over 24 hours.
FIG. 17 shows shows the drug release profile of Compound A in Formulations 92 to 93 over 24 hours.
FIG. 18 shows shows the drug release profile of Compound A in bilayer tablets over 24 hours.
FIG. 19 shows the drug release profile of Compound A in Formulations 94 to 96 over 24 hours.

### 3.2. DEFINITIONS

As used herein, the term "patient" refers to a mammal, particularly a human.

As used herein, the term "pharmaceutically acceptable salts" refer to salts prepared from pharmaceutically acceptable non-toxic acids or bases including inorganic acids and bases and organic acids and bases.

As used herein and unless otherwise indicated, the term "prodrug" means a derivative of a compound that can hydrolyze, oxidize, or otherwise react under biological conditions (*in vitro* or *in vivo*) to provide the compound. Examples of prodrugs include, but are not limited to, derivatives and metabolites of a compound provided herein that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Prodrugs can typically be prepared using well-known methods, such as those described by 1 Burger's Medicinal Chemistry and Drug Discovery, 172-178, 949-982 (Manfred E. Wolff ed., 5th ed. 1995).

As used herein and unless otherwise indicated, the terms "biohydrolyzable amide," "biohydrolyzable ester," "biohydrolyzable carbamate," "biohydrolyzable carbonate," "biohydrolyzable ureide," "biohydrolyzable phosphate" mean an amide, ester, carbamate, carbonate, ureide, or phosphate, respectively, of a compound that either: 1) does not interfere with the biological activity of the compound but can confer upon that compound advantageous properties in vivo, such as uptake, duration of action, or onset of action; or 2) is biologically inactive but is converted in vivo to the biologically active compound. Examples of biohydrolyzable esters include, but are not limited to, lower alkyl esters, alkoxyacyloxy esters, alkyl acylamino alkyl esters, and choline esters. Examples of biohydrolyzable amides include, but are not limited to, lower alkyl amides, α-amino acid amides, alkoxyacyl amides, and alkylaminoalkylcarbonyl amides. Examples of biohydrolyzable carbamates include, but are not limited to, lower alkylamines, substituted ethylenediamines, aminoacids, hydroxyalkylamines, heterocyclic and heteroaromatic amines, and polyether amines.

As used herein and unless otherwise indicated, the term "stereomerically pure" means a composition that comprises one stereoisomer of a compound and is substantially free of other stereoisomers of that compound. For example, a stereomerically pure composition of a compound having one chiral center will be substantially free of the opposite enantiomer of the compound. A stereomerically pure composition of a compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound.

As used herein and unless otherwise indicated, the term "enantiomerically pure" means a stereomerically pure composition of a compound having one chiral center.

As used herein, term "adverse effects" includes, but is not limited to gastrointestinal, renal and hepatic toxicities, leukopenia, increases in bleeding times due to, *e.g*., thrombocytopenia, and prolongation of gestation, nausea, vomiting, somnolence, asthenia, dizziness, teratogenicity, extra-pyramidal symptoms, akathisia, cardiotoxicity including cardiovascular disturbances, inflammation, male sexual dysfunction, and elevated serum liver enzyme levels. The term "gastrointestinal toxicities" includes but is not limited to gastric and intestinal ulcerations and erosions. The term "renal toxicities" includes but is not limited to such conditions as papillary necrosis and chronic interstitial nephritis.

As used herein and unless otherwise indicated, the phrases "reduce or avoid adverse effects" and "reducing or avoiding adverse effects" mean the reduction of the severity of one or more adverse effects as defined herein.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

As used herein and unless otherwise specified, the term "crystalline" and related terms used herein, when used to describe a compound, substance, modification, material, component or product, unless otherwise specified, mean that the compound, substance, modification, material, component or product is substantially crystalline as determined by X-ray diffraction. *See, e.g.,* Remington: The Science and Practice of Pharmacy, 21st edition, Lippincott, Williams and Wilkins, Baltimore, MD (2005); The United States Pharmacopeia, 23rd ed., 1843-1844 (1995).

As used herein and unless otherwise specified, the term "crystal forms," "crystalline forms" and related terms herein refer to solid forms that are crystalline. Crystal forms include single-component crystal forms and multiple-component crystal forms, and include, but are not limited to, polymorphs, solvates, hydrates, and/or other molecular complexes. In certain embodiments, a crystal form of a substance may be substantially free of amorphous forms and/or other crystal forms. In certain embodiments, a crystal form of a substance may contain less than about 1%, 2%, 3%, 4%, 5%, 6%, 7%, 8%, 9%, 10%, 15%, 20%, 25%, 30%, 35%, 40%, 45% or 50% of one or more amorphous forms and/or other crystal forms on a weight basis. In certain embodiments, a crystal form of a substance may be physically and/or chemically pure. In certain embodiments, a crystal form of a substance may be about 99%, 98%, 97%, 96%, 95%, 94%, 93%, 92%, 91% or 90% physically and/or chemically pure.

As used herein and unless otherwise specified, the terms "solvate" and "solvated," refer to a crystal form of a substance which contains solvent. The terms "hydrate" and "hydrated" refer to a solvate wherein the solvent comprises water. "Polymorphs of solvates" refers to the existence of more than one crystal form for a particular solvate composition. Similarly, "polymorphs of hydrates" refers to the existence of more than one crystal form for a particular hydrate composition. The term "desolvated solvate," as used herein, refers to a crystal form of a substance which may be prepared by removing the solvent from a solvate.

As used herein and unless otherwise specified, the terms "about" and "approximately," when used in connection with a numeric value or a range of values which is provided to characterize a particular solid form, *e.g.,* a specific temperature or temperature range, such as, *e.g.,* that describing a DSC or TGA thermal event, including, *e.g.,* melting, dehydration, desolvation or glass transition events; a mass change, such as, *e.g.,* a mass change as a function of temperature or humidity; a solvent or water content, in terms of, *e.g.,* mass or a percentage; or a peak position, such as, *e.g.,* in analysis by IR or Raman spectroscopy or XRPD; indicate that the value or range of values may deviate to an extent deemed reasonable to one of ordinary skill in the art while still describing the particular solid form. For example, in particular embodiments, the terms "about" and "approximately," when used in this context and unless otherwise specified, indicate that the numeric value or range of values may vary within 25%, 20%, 15%, 10%, 9%, 8%, 7%, 6%, 5%, 4%, 3%, 2%, 1.5%, 1%, 0.5%, or 0.25% of the recited value or range of values.

As used herein and unless otherwise specified, a sample comprising a particular crystal form or amorphous form that is "substantially pure," *e.g*., substantially free of other solid forms and/or of other chemical compounds, contains, in particular embodiments, less than about 25%, 20%, 15%, 10%, 9%, 8%, 7%, 6%, 5%, 4%, 3%, 2%, 1%, 0.75%, 0.5%, 0.25% or 0.1% percent by weight of one or more other solid forms and/or of other chemical compounds.

As used herein and unless otherwise specified, a sample or composition that is "substantially free" of one or more other solid forms and/or other chemical compounds means that the composition contains, in particular embodiments, less than about 25%, 20%, 15%, 10%, 9%, 8%, 7%, 6%, 5%, 4%, 3%, 2%, 1%, 0.75%, 0.5%, 0.25% or 0.1% percent by weight of one or more other solid forms and/or other chemical compounds.

As used herein, and unless otherwise specified, the terms "treat," "treating" and "treatment" refer to the eradication or amelioration of a disease or disorder, or of one or more symptoms associated with the disease or disorder. In certain embodiments, the terms refer to minimizing the spread or worsening of the disease or disorder resulting from the administration of one or more prophylactic or therapeutic agents to a patient with such a disease or disorder. In some embodiments, the terms refer to the administration of a compound provided herein, with or without other additional active agent, after the onset of symptoms of the particular disease.

As used herein, and unless otherwise specified, the terms "prevent," "preventing" and "prevention" refer to the prevention of the onset, recurrence or spread of a disease or disorder, or of one or more symptoms thereof. In certain embodiments, the terms refer to the treatment with or administration of a compound provided herein, with or without other additional active compound, prior to the onset of symptoms, particularly to patients at risk of diseases or disorders provided herein. The terms encompass the inhibition or reduction of a symptom of the particular disease. Patients with familial history of a disease in particular are candidates for preventive regimens in certain embodiments. In addition, patients who have a history of recurring symptoms are also potential candidates for the prevention. In this regard, the term "prevention" may be interchangeably used with the term "prophylactic treatment."

As used herein, and unless otherwise specified, the terms "manage," "managing" and "management" refer to preventing or slowing the progression, spread or worsening of a disease or disorder, or of one or more symptoms thereof. Often, the beneficial effects that a patient derives from a prophylactic and/or therapeutic agent do not result in a cure of the disease or disorder. In this regard, the term "managing" encompasses treating a patient who had suffered from the particular disease in an attempt to prevent or minimize the recurrence of the disease.

As used herein, and unless otherwise specified, a "therapeutically effective amount" of a compound is an amount sufficient to provide a therapeutic benefit in the treatment or management of a disease or disorder, or to delay or minimize one or more symptoms associated with the disease or disorder. A therapeutically effective amount of a compound means an amount of therapeutic agent, alone or in combination with other therapies, which provides a therapeutic benefit in the treatment or management of the disease or disorder. The term "therapeutically effective amount" can encompass an amount that improves overall therapy, reduces or avoids symptoms or causes of disease or disorder, or enhances the therapeutic efficacy of another therapeutic agent.

As used herein, and unless otherwise specified, a "prophylactically effective amount" of a compound is an amount sufficient to prevent a disease or disorder, or prevent its recurrence. A prophylactically effective amount of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

The term "composition" as used herein is intended to encompass a product comprising the specified ingredients (and in the specified amounts, if indicated), as well as any product which results, directly or indirectly, from combination of the specified ingredients in the specified amounts. By "pharmaceutically acceptable" it is meant that the diluent, excipient or carrier must be compatible with the other ingredients of the formulation and not deleterious to the recipient thereof.

### 4. DETAILED DESCRIPTION

Provided herein are controlled release oral dosage forms of the poorly soluble drug of formula (I) as defined in the claims, methods of making the solid forms, and methods of their use for the treatment of various diseases and/or disorders.

The controlled release oral dosage forms provided herein comprise polymeric excipients which expand and/or become charged in the gastric fluid in acidic pH and control the release of the poorly soluble drug in the system.

Without being bound to a particular theory, the controlled release oral dosage forms provided herein are believed to enhance the bioavailability of a poorly soluble drug by increasing the time of release of the drug in the gastrointestinal tract. In some embodiments, the extended time of release of the poorly soluble drug occurs mainly in the stomach.

In certain embodiments, the release profile of the dosage forms provided herein achieve controlled release over an 8 to 24 hour period. In some embodiments, controlled release is achieved over about an 8 hour period; a 10 hour period; a 12 hour period; a 14 hour period; a 16 hour period; an 18 hour period; a 20 hour period; a 22 hour period; or a 24 hour period.

In some embodiments, the controlled release oral dosage forms provided herein comprise positively charged polymers, negatively charged polymers and swelling excipients, which when combined with a poorly soluble drug in particular weight ratios of ingredients provide controlled release of the poorly soluble drug. Without being bound to a particular theory, controlled release of the poorly soluble drug is achieved by action of the swelling excipients and the interaction of the polymers containing negative charges and positive charges in acidic pH of the stomach or upper gastrointestinal tract.

In one embodiment, the controlled release oral dosage form comprises the following: (i) the poorly soluble drug of formula (I); (ii) a swelling excipient; (iii) a cationic polymer in acidic pH; and (iv) an anionic polymer in acidic pH as defined in the claims. In some embodiments, the controlled release oral dosage form further comprises a water absorbing agent. In some embodiments, the controlled release oral dosage form further comprises one or more additional pharmaceutically acceptable excipients.

As provided herein, a "cationic polymer in acidic pH" or "positively charged polymer" refers to a polymer which is positively charged in acidic pH. "Acidic pH" refers to a pH < 7. In some embodiments "acid pH" refers to a pH between 0 and 7; 0 and 5; 1 and 5; 0 and 4; 1 and 4; 0 and 3; or 1 and 3. The cationic polymer in acidic pH is selected from the group consisting of chitosan (*e.g*., Chitopharm^{®} S and Chitoclear^{®} 2832, 3504, 3548 and 3568), methacrylic acid - methyl methacrylate copolymer (1:1) (Eudragit^{®} L100, Eudragit^{®} L100-55), methacrylic acid - methyl methacrylate copolymer (1:2) (Eudragit^{®} S 100), poly(butyl methacylate-co-2-dimethylaminoethyl methacrylate-co-methyl methacrylate) (1:2:1) (Eudragit^{®} E PO), Eudragit^{®} R LPO, Eudragit^{®} R SPO, and crosslinked acrylic acid copolymers (Car-bopol^{®}).

As provided herein, an "anionic polymer in acidic pH" or "negatively charged polymer" refers to a polymer which is negatively charged in acidic pH. The anionic polymer in acidic pH is selected from the group consisting of sodium alginate (e.g., Protanal^{®} LF 120M, Protanal^{®} LF 200M, Protanal^{®} LF 200D), sodium carboxymethyl cellulose (CMC), chondroitin sulfate, carrageenan (*e.g.,* Gelcarin^{®} 209, Gelcarin^{®} 379), glycosaminoglycans, mucopolysaccharides, pectin, gelatin and hyaleuronic acid.

As provided herein, a "swelling excipient" refers to an excipient which swells or grows in size when in contact with a liquid, *e.g.,* an aqueous solution. The swelling excipient is selected from hydroxyethylcellulose (HEC, *e.g*., Natrosol^{®} G, Natrosol^{®} L), polyethylene oxide (*e.g*., Polyox^{®} N10, Polyox^{®} N12K, Polyox^{®} N80, Polyox^{®} N-205G, Polyox^{®} N-1105 and Polyox^{®} N750), sodium carboxymethyl cellulose (CMC, *e.g*., CMC 7L2P and CMC 7LF), hydroxypropyl cellulose, hydroxylpropyl methyl cellulose (HPMC), methyl celluloses, sodium crosscarmellose (Ac-Di-Sol^{®}), and sodium starch glycolate (Primojel^{®}).

Nonlimiting examples of water absorbing agent include humectants such as sorbitol, xylitol, maltitol, polymeric polyols, calcium chloride, sodium chloride, carrageenan (Gelcarin^{®}), polyacrylic acid and hydrogel.

Fillers and processing aids may be used in the controlled release dosage forms provided herein. Examples of fillers include, but are not limited to, microcrystalline cellulose (*e.g.,* MCC, Avicel PH102), lactose, dicalcium phosphate, pregelatinized starch and the mixture thereof.

Surfactants may be used in the controlled release dosage forms provided herein. Examples of surfactants include, but are not limited to, sodium laural sulfate (SLS) and ethylene oxide - propylene oxide block copolymers (*e.g*., Pluronic^{®} F108).

As provided herein, "poorly soluble drug" refers to a drug which has limited solubility in aqueous media. Poorly soluble drugs are not readily absorbed through the gastrointestinal tract upon oral administration.

The poorly soluble drug provided herein is (*S*)-*N*-{2-[1-(3-ethoxy-4-methoxy-phenyl)-2-methanesulfonylethyl]-1,3-dioxo-2,3-dihydro-1*H*-isoindol-4-yl}acetamide (Compound A).

(*S*)-*N*-{2-[1-(3-ethoxy-4-methoxy-phenyl)-2-methanesulfonylethyl]-1,3-dioxo-2,3-dihydro-1*H*-isoindol-4-yl} acetamide (Compound A), described in Man et al. J. Med. Chem., 2009, 52, 1522-1524, has the following structure:

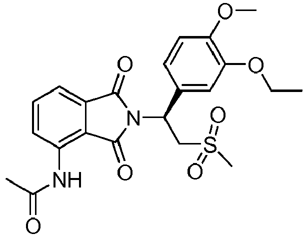

Another soluble drug described herein is cyclopropanecarboxylic acid {2-[(*1S*)-1-(3-ethoxy-4-methoxy-phenyl)-2-methanesulfonyl-ethyl]-3-oxo-2,3-dihydro-1*H*-isoindol-4-yl}-amide (Compound B) which has the following structure:

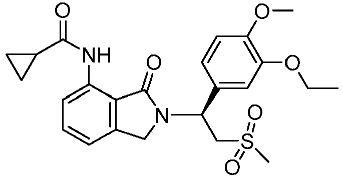

The aqueous room temperature solubilities of Compounds A and Compound B is 6.9 µg/mL and 2.0 µg/mL, respectively. Daily doses of Compound A ranging from 10 mg to 100 mg per day have been administered to approximately 1000 subject in clinical studies to date. At dose 10 mg/kg PO, the pharmacokinetic parameters of Compound A in monkeys indicated that the t_{1/2} is about 2 hours. Therefore a controlled release dosage is clearly needed for Compound A.

In one embodiment, the controlled release oral dosage form comprises a poorly soluble drug, chitosan, an alginate, a swelling excipient, and optionally one or more additional excipients. In one embodiment, the swelling polymer is Natrosol. In one embodiment, the swelling polymer is Polyox.

In one embodiment, the chitosan has an average molecular weight of 10,000 to 5,000,000 Da. In another embodiment, the chitosan has an average molecular weight of 10,000 to 2,000,000 Da. In some embodiments, the chitosan has a degree of deacylation of at least 70%. In other embodiments, the chitosan has a degree of deacylation of at least 90%. In one embodiment, the particle size of the chitosan is such that it passes through 20 mesh screen.

In one embodiment, the alginate is a salt of aginic acid. In one embodiment, the alginate is sodium alginate.

In one embodiment, the controlled release oral dosage form comprises a poorly soluble drug, chitosan, a salt of carboxymethyl cellulose, a swelling excipient, and optionally one or more additional excipients.

In some embodiments, the swelling excipient is a polyethylene oxide or hydroxyethyl cellulose.

In some embodiments, the controlled release oral dosage form further comprises a disintegrant. In certain embodiments, the disintegrant is lactose. In other embodiments, the disintegrant is microcrystalline cellulose (MCC). In other embodiments, the disintegrant is sodium crosscarmellose. In other embodiments, the disintegrant is Primojel^{®}.

Provided herein are methods of treating, preventing or managing disorders ameliorated by the reduction of levels of TNF-α in a patient which comprises administering to a patient in need of such treatment, prevention or management a therapeutically or prophylactically effective amount of a compound provided herein, or a pharmaceutically acceptable polymorph, solvate, or hydrate thereof.

In particular embodiments, diseases or disorders ameliorated by the inhibition of TNF-α production in mammals include, but are not limited to: HIV; hepatitis; adult respiratory distress syndrome; bone resorption diseases; chronic obstructive pulmonary diseases; chronic pulmonary inflammatory diseases; asthma; dermatitis; cystic fibrosis; septic shock; sepsis; endotoxic shock; hemodynamic shock; sepsis syndrome; post ischemic reperfusion injury; meningitis; psoriasis; psoriatic arthritis; ankylosing spondylitis; Behcet's Disease; fibrotic disease; cachexia; graft rejection; auto immune disease; rheumatoid spondylitis; arthritic conditions, such as psoriatic arthritis, rheumatoid arthritis and osteoarthritis; osteoporosis; Crohn's disease; ulcerative colitis; inflammatory bowel disease; multiple sclerosis; systemic lupus erythematosus; cutaneous lupus erythematosus; pulmonary sarcoidosis; erythema nodosum leprosum (ENL) in leprosy; radiation damage; asthma; and hyperoxic alveolar injury. Such disorders further include, but are not limited to, cancers, including, but not limited to cancer of the head, thyroid, neck, eye, skin, mouth, throat, esophagus, chest, bone, blood, bone marrow, lung, colon, sigmoid, rectum, stomach, prostate, breast, ovaries, kidney, liver, pancreas, brain, intestine, heart, adrenal, subcutaneous tissue, lymph nodes, heart, and combinations thereof. Specific cancers that can be treated by this method are multiple myeloma, malignant melanoma, malignant glioma, leukemia and solid tumors.

In some embodiments, provided herein are methods of treating or preventing cancer, including but not limited to, solid tumor, blood-borne tumor, leukemias, and in particular, multiple myeloma in a patient which comprises administering to a patient in need of such treatment or prevention a therapeutically effective amount of a compound provided herein, or a pharmaceutically acceptable prodrug, metabolite, polymorph, solvate, hydrate, or clathrate thereof; in particular wherein the patient is a mammal.

In another embodiment provided herein is a method of inhibiting PDE4 which comprises contacting PDE4 in a cell (*e.g.* a mammalian cell) with an effective amount of a compound provided herein, or a pharmaceutically acceptable polymorph, solvate, or hydrate thereof (wherein particular embodiments encompass solid forms comprising Compound A as described herein).

In further embodiments, provided herein are methods of treating or preventing diseases or disorders ameliorated by the inhibition of PDE4 in a patient which comprises administering to a patient in need of such treatment or prevention a therapeutically or prophylactically effective amount of a compound provided herein, or a pharmaceutically acceptable polymorph, solvate, or hydrate thereof. Disorders ameliorated by the inhibition of PDE4 include, but are not limited to, asthma, inflammation (*e.g*., inflammation due to reperfusion), chronic or acute obstructive pulmonary diseases, chronic or acute pulmonary inflammatory diseases, cutaneous lupus erythematosis, inflammatory bowel disease, Crohn's Disease, Behcet's Disease, or colitis.

In other embodiments, provided herein are methods of controlling cAMP levels in a cell which comprises contacting a cell with an effective amount of a compound provided herein, or a pharmaceutically acceptable polymorph, solvate, or hydrate thereof. As used herein the term "controlling cAMP levels" includes preventing or reducing the rate of the breakdown of Adenosine 3',5'-cyclic monophosphate (cAMP) in a cell or increasing the amount of Adenosine 3',5'-cyclic monophosphate present in a cell, preferably a mammalian cell, more preferably a human cell. In a particular method, the rate of cAMP breakdown is reduced by about 10, 25, 50, 100, 200, or 500 percent as compared to the rate in comparable cells which have not been contacted with a compound of the invention.

In other embodiments, provided herein are methods of treating or preventing depression, asthma, inflammation, contact dermatitis, atopic dermatitis, psoriasis, psoriatic arthritis, rheumatoid arthritis, osteoarthritis, cutaneous lupus erythematosis, ankylosing spondylitis, inflammatory skin disease, inflammation due to reperfusion, chronic or acute obstructive pulmonary diseases, chronic or pulmonary inflammatory diseases, autoimmune diseases, inflammatory bowel disease, Crohn's Disease, Behcet's Disease or colitis in a patient which comprises administering to a patient in need of such treatment or prevention a therapeutically or prophylactically effective amount of a compound provided herein, or a pharmaceutically acceptable polymorph, solvate, or hydrate thereof; in particular wherein the patient is a mammal.

In other embodiments, provided herein are methods of treating or preventing myelodysplastic syndrome (MDS) which comprises administering to a patient in need of such treatment or prevention a therapeutically or prophylactically effective amount of a compound provided herein, or a pharmaceutically acceptable solvate or hydrate thereof. MDS refers to a diverse group of hematopoietic stem cell disorders. MDS is characterized by a cellular marrow with impaired morphology and maturation (dysmyelopoiesis), peripheral blood cytopenias, and a variable risk of progression to acute leukemia, resulting from ineffective blood cell production. *See* The Merck Manual 953 (17th ed. 1999) and List et al., 1990, J. Clin. Oncol. 8:1424.

Also provided herein are methods of treating or preventing myeloproliferative disease (MPD) which comprises administering to a patient in need of such treatment or prevention a therapeutically or prophylactically effective amount of a compound provided herein, or a pharmaceutically acceptable solvate or hydrate thereof. Myeloproliferative disease (MPD) refers to a group of disorders characterized by clonal abnormalities of the hematopoietic stem cell. *See e.g.,* Current Medical Diagnosis & Treatment, pp. 499 (37th ed., Tierney et al., ed., Appleton & Lange, 1998).

Also provided herein are methods of treating, preventing or managing pain, including, but not limited to, complex regional pain syndrome, which comprises administering to a patient in need of such treatment, prevention or management a therapeutically or prophylactically effective amount of a compound provided herein, or a pharmaceutically acceptable solvate or hydrate thereof. In a specific embodiment, the administration is before, during or after surgery or physical therapy directed at reducing or avoiding a symptom of complex regional pain syndrome in the patient.

In some methods herein, a compound provided herein, or a pharmaceutically acceptable polymorph, solvate or hydrate thereof, is adjunctively administered with at least one additional therapeutic agent. Examples of additional therapeutic agents include, but are not limited to, anti-cancer drugs, antiinflammatories, antihistamines and decongestants.

### 4.1. CONTROLLED RELEASE ORAL DOSAGE FORMS

The controlled release dosage forms provided herein comprise positively charged polymers, negatively charged polymers and swelling excipients, which when combined with a poorly soluble drug in particular weight ratios of ingredients provide controlled release of the poorly soluble drug. Without being bound to a particular theory, controlled release of the poorly soluble drug is achieved by action of the swelling excipients and the interaction of the polymers containing negative charges and positive charges in acidic pH of the stomach or upper gastrointestinal tract.

In certain embodiments, the release profile of the dosage forms provided herein achieves controlled release over an 8 to 24 hour period.

The controlled release dosage forms provided herein use opposite charged polymeric excipients to form an inter-penetrating network *in situ* when the compositions contact water, gradually forming a gel system in the outer shell of the dosage form (*e.g.,* tablet). A water absorbing agent enhances the rate of water penetration to boost the swelling of the inter-penetrating system in a short time. Furthermore, specific excipients which contribute to the swelling result in a synergistic swelling ratio with the charged inter-penetrating network system.

Controlled release oral dosage forms provided herein comprise the following: (i) the poorly soluble drug of formula (I); (ii) a swelling excipient; (iii) a cationic polymer in acidic pH; and (iv) an anionic polymer in acidic pH as defined in the claims. In some embodiments, the controlled release oral dosage form further comprises a water absorbing agent. In some embodiments, the controlled release oral dosage form further comprises one or more additional pharmaceutically acceptable excipients.

Only certain pH-sensitive polymers combined with swelling excipients can achieve a beneficial gastroretentive systems provided herein. In the controlled release dosage forms provided herein, specific polymers are selected which bear positive and negative charges at the pH of the stomach, and with specific swelling ingredients, the systems show the gastroretentive effects by swelling the matrix for extended controlled release of a drug or drugs. Further, in some embodiments, the rates of release of drugs from the system may be controlled by altering the ratio of ingredients, *e.g*., of charged polymers and swelling excipients. The ranges of molecular weight of polymers in the inter-penetrating system also may contribute to the controlled release pattern of the drugs.

Chitosans are exemplary positively charged polymers that may be used in the oral dosage forms provided herein. Chitosans have been described in the literature as pharmaceutical ingredients for controlled release systems. *See e.g.,* Eur J Pharm Sci., 2003, 19(5):345-53. However, the use of chitosans is limited to controlled release systems for drug delivery in the colon, not the gastroretentive system. The gastric retention time described in these systems is too short; drugs pass the absorption window in stomach before being released.

As provided herein, the molecular weight, particle size, and degree of deacetylation of chitosans are factors which may affect release rates and lengthen the widow of absorption of a drug. In some embodiments, the degree of deacetylation of chitosans used in the formulations herein is greater than 90%. In the prior art, chitosan was prepared by dissolving the granules in acid solution first, followed by drying to lumps and homogenized. As provided herein, chitosan granules are used directly without re-processing.

The controlled release dosage forms provided herein are developed such that the amount of the total excipients required for swelling and retaining in the stomach over time is determined such that the system delivers the drug in a controlled release manner. Certain combinations of positively charged polymer (*e.g*., chitosan), negatively charged polymer (*e.g*. sodium alginate) and swelling ingredients (*e.g*., Ac-Di-Sol^{®} or Natrosol^{®}) are proved to be a synergistic controlled release system. Such compositions result in extended controlled release profiles by USP I *in vitro* dissolution method using Distek dissolution apparatus. *See* Examples 4-6.

### 4.2. PHARMACEUTICAL COMPOSITIONS

Pharmaceutical compositions and dosage forms provided herein typically also comprise one or more pharmaceutically acceptable excipient, diluent or carrier.

In some embodiments, a pharmaceutical composition provided herein comprises one or more solid forms a compound provided herein and at least one additional therapeutic agent. Examples of additional therapeutic agents include, but are not limited to: anti-cancer drugs and anti-inflammation therapies including, but not limited to, those provided herein.

Examples of oral dosage forms include, but are not limited to: tablets; caplets; capsules, such as soft elastic gelatin capsules; cachets; troches; lozenges; dispersions; aerosols (*e.g*., inhalers); gels; liquid dosage forms suitable for oral administration to a patient, including suspensions (*e.g*., aqueous or non-aqueous liquid suspensions, oil-in-water emulsions, or a water-in-oil liquid emulsions), solutions, and elixirs.

The composition, shape, and type of dosage forms provided herein will typically vary depending on their use. These variations will be readily apparent to those skilled in the art. *See, e.g.,* Remington's Pharmaceutical Sciences, 18th ed., Mack Publishing, Easton PA (1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form.

Lactose-free compositions of the invention can comprise excipients that are well known in the art and are listed, for example, in the U.S. Pharmocopia (USP) SP (XXI)/NF (XVI). In general, lactose-free compositions comprise an active ingredient, a binder/filler, and a lubricant in pharmaceutically compatible and pharmaceutically acceptable amounts. Preferred lactose-free dosage forms comprise an active ingredient, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water (*e.g.,* 5%) is widely accepted in the pharmaceutical arts as a means of simulating long-term storage in order to determine characteristics such as shelf-life or the stability of formulations over time. *See, e.g.,* Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

The dosage forms provided herein may further comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms provided herein lie within the range of from about 1 mg to about 1,000 mg per day, given as a single once-a-day dose in the morning but preferably as divided doses throughout the day. More specifically, the daily dose is administered twice daily in equally divided doses. Specifically, a daily dose range may be from about 5 mg to about 500 mg per day, more specifically, between about 10 mg and about 200 mg per day. In managing the patient, the therapy may be initiated at a lower dose, perhaps about 1 mg to about 25 mg, and increased if necessary up to about 200 mg to about 1,000 mg per day as either a single dose or divided doses, depending on the patient's global response.

The oral dosage forms provided herein may be presented as discrete dosage forms, such as, but are not limited to, tablets (*e.g*., chewable tablets), caplets, capsules, and liquids (*e.g*., flavored syrups). Such dosage forms contain predetermined amounts of active ingredients, and may be prepared by methods of pharmacy well known to those skilled in the art. *See generally* Remington's Pharmaceutical Sciences, 18th ed., Mack Publishing, Easton PA (1990).

Typical oral dosage forms provided herein are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.,* powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

In certain embodiments, the dosage form provided herein is a 5 mg, 10 mg, 15 mg, 20 mg, 25 mg, 30 mg, 40 mg, 50 mg, 60 mg, 75 mg, 100 mg, 150 mg, 200 mg, 250 mg, 300 mg, 400 mg, 500 mg, 750 mg or 1000 mg tablet.

Examples of excipients that can be used in oral dosage forms provided herein include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g.,* Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Suitable forms of microcrystalline cellulose include, but are not limited to, the materials sold as AVICEL-PH-101^{™}, AVICEL-PH-103^{™}, AVICEL RC-581^{™}, AVICEL-PH-105^{™} (available from FMC Corporation, American Viscose Division, Avicel Sales, Marcus Hook, PA), and mixtures thereof. A specific binder is a mixture of microcrystalline cellulose and sodium carboxymethyl cellulose (sodium CMC) sold, for example, as AVICEL RC-581^{™}. Suitable anhydrous or low moisture excipients or additives include AVICEL-PH-103^{™} and Starch 1500 LM^{™}.

Disintegrants may be used in the compositions herein to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that may be used herein include, but are not limited to, agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Lubricants that may be used herein include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200^{™}, manufactured by W.R. Grace Co. of Baltimore, MD), a coagulated aerosol of synthetic silica (marketed by Degussa Co. of Plano, TX), CAB-O-SIL^{™} (a pyrogenic silicon dioxide product sold by Cabot Co. of Boston, MA), and mixtures thereof. If used at all, lubricants are typically used in an amount of less than about one weight percent of the pharmaceutical compositions or dosage forms into which they are incorporated.

Dosage forms comprising a compound may be administered by controlled release means or by delivery devices that are well known to those of ordinary skill in the art. Examples include, but are not limited to, those described in U.S. Patent Nos.: 3,536,809; 3,598,123; 3,845,770; 3,916,899; 4,008,719; 5,059,595; 5,073,543; 5,120,548; 5,354,556; 5,591,767; 5,639,476; 5,674,533 and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### 4.3. METHODS OF TREATMENT

The invention encompasses methods of treating, preventing and managing diseases or disorders ameliorated by the reduction of levels of TNF-α in a patient which comprise administering to a patient in need of such treatment, prevention or management a therapeutically or prophylactically effective amount of a controlled release oral dosage form provided herein.

Disorders ameliorated by the inhibition of TNF-α include, but are not limited to: heart disease, such as congestive heart failure, cardiomyopathy, pulmonary edema, endotoxin-mediated septic shock, acute viral myocarditis, cardiac allograft rejection, and myocardial infarction; depression, asthma, inflammation, contact dermatitis, atopic dermatitis, psoriasis, psoriatic arthritis, rheumatoid arthritis, osteoarthritis, cutaneous lupus erythematosis, ankylosing spondylitis, inflammatory skin disease, inflammation due to reperfusion, chronic or acute obstructive pulmonary diseases, chronic or pulmonary inflammatory diseases, autoimmune diseases, inflammatory bowel disease, Crohn's Disease, Behcet's Disease or colitis; solid tumors, including but not limited to, sarcoma, carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, Kaposi's sarcoma, pinealoma, hemangioblastoma, acoustic neuroma, oligodendroglioma, menangioma, melanoma, neuroblastoma, and retinoblastoma; and blood-borne tumors including but not limited to, acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", hairy cell leukemia, multiple myeloma and acute and chronic leukemias, for example, lymphoblastic, myelogenous, lymphocytic, and myelocytic leukemias.

Specific methods provided herein further comprise the administration of an additional therapeutic agent. Examples of additional therapeutic agents include, but are not limited to, anti-cancer drugs such as, but are not limited to: alkylating agents, nitrogen mustards, ethylenimines, methylmelamines, alkyl sulfonates, nitrosoureas, triazenes, folic acid analogs, pyrimidine analogs, purine analogs, vinca alkaloids, epipodophyllotoxins, antibiotics, topoisomerase inhibitors and anti-cancer vaccines.

Specific additional therapeutic agents include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1 ; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

Embodiments herein further encompass a method of treating or preventing diseases or disorders ameliorated by the inhibition of TNF-α in a patient. Such diseases and disorders include, but are not limited to: heart disease, such as congestive heart failure, cardiomyopathy, pulmonary edema, endotoxin-mediated septic shock, acute viral myocarditis, cardiac allograft rejection, and myocardial infarction; depression, asthma, inflammation (*e.g*., contact dermatitis, atopic dermatitis, psoriasis, psoriatic arthritis, rheumatoid arthritis, osteoarthritis, cutaneous lupus erythematosis, ankylosing spondylitis, inflammatory skin disease, inflammation due to reperfusion), chronic or acute obstructive pulmonary diseases, chronic or pulmonary inflammatory diseases, autoimmune diseases, inflammatory bowel disease, Crohn's Disease, Behcet's Disease or colitis. In a one embodiment, the disease or disorder to be treated or prevented is chronic obstructive pulmonary disease.

Specific methods provided herein may comprise the administration of an additional therapeutic agent such as, but not limited to, anti-inflammatory drugs, antihistamines and decongestants. Examples of such additional therapeutic agents include, but are not limited to: antihistamines including, but not limited to, ethanolamines, ethylenediamines, piperazines, and phenothiazines; antinflammatory drugs; NSAIDS, including, but not limited to, aspirin, salicylates, acetominophen, indomethacin, sulindac, etodolac, fenamates, tolmetin, ketorolac, diclofenac, ibuprofen, naproxen, fenoprofen, ketoprofen, flurbiprofen, oxaprozin, piroxicam, meloxicam, pyrazolon derivatives; and steriods including, but not limited to, cortical steroids and adrenocortical steroids.

As stated above, the dosage forms provided herein may be used in the treatment or prevention of a wide range of diseases and conditions. The magnitude of a prophylactic or therapeutic dose of a particular active ingredient of the invention in the acute or chronic management of a disease or condition may vary with the nature and severity of the disease or condition and the route by which the active ingredient is administered. The dose, and perhaps the dose frequency, will also vary according to the age, body weight, and response of the individual patient. Suitable dosing regimens can be readily selected by those skilled in the art with due consideration of such factors. In general, the recommended daily dose range for the conditions described herein lie within the range of from about 1 mg to about 1,000 mg per day, given as a single once-a-day dose preferably as divided doses throughout a day. More specifically, the daily dose is administered twice daily in equally divided doses. Specifically, a daily dose range may be from about 5 mg to about 500 mg per day, more specifically, between about 10 mg and about 200 mg per day. Specifically, the daily dose may be administered in 5 mg, 10 mg, 15 mg, 20 mg, 25 mg, 30 mg, 50 mg, or 100 mg dosage forms (Q.D. or B.I.D.). In managing the patient, the therapy should be initiated at a lower dose, perhaps about 1 mg to about 25 mg, and increased if necessary up to about 200 mg to about 1,000 mg per day as either a single dose or divided doses, depending on the patient's global response. Alternatively, the daily dose is from 0.01 mg/kg to 100 mg/kg.

It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with individual patient response.

### 4.3.1. KITS

This invention encompasses kits which, when used by the medical practitioner, can simplify the administration of appropriate amounts of active ingredients to a patient.

A typical kit of the invention comprises a unit dosage form of a compound provided herein, or a pharmaceutically acceptable solid form or prodrug thereof, and a unit dosage form of a second active ingredient. Examples of second active ingredients include, but are not limited to, those listed herein.

Kits of the invention can further comprise devices that are used to administer the active ingredient(s). Examples of such devices include, but are not limited to, syringes, drip bags, patches, and inhalers.

Kits of the invention can further comprise pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### 5. EXAMPLES

### 5.1. EXAMPLE 1 (not in the scope of the claims): SYNTHESIS OF 2-[1-(3-ETHOXY-4-METHOXYPHENYL)-2-METHYLSULFONYLETHYL]-4-ACETYLAMINOISOINDOLINE-1,3-DIONE

A stirred solution of 1-(3-ethoxy-4-methoxyphenyl)-2-methylsulfonylethylamine (1.0 g, 3.7 mmol) and 3-acetamidophthalic anhydride (751 mg, 3.66 mmol) in acetic acid (20 mL) was heated at reflux for 15 h. The solvent was removed *in vacuo* to yield an oil. Chromatography of the resulting oil yielded the product as a yellow solid (1.0 g, 59% yield): mp, 144 °C; ¹H NMR (CDCl₃) δ: 1.47 (t, J=7.0 Hz, 3H, CH₃), 2.26 (s, 3H, CH₃), 2.88 (s, 3H, CH₃), 3.75 (dd, J=4.4, 14.3 Hz, 1H, CH), 3.85 (s, 3H, CH3), 4.11 (q, J=7 Hz, 2H, CH₂), 5.87 (dd, J=4.3, 10.5 Hz, 1H, NCH), 6.82-6.86 (m, 1H, Ar), 7.09-7.11 (m, 2H, Ar), 7.47 (d, J= 7 Hz, 1H, Ar), 7.64 (t, J= 8 Hz, 1H, Ar), 8.74 (d, J= 8 Hz, 1H, Ar), 9.49 (br s, 1H, NH); ¹³C NMR (CDCl₃) δ: 14.61, 24.85, 41.54, 48.44, 54.34, 55.85, 64.43, 111.37, 112.34, 115.04, 118.11, 120.21, 124.85, 129.17, 130.96, 136.01, 137.52, 148.54, 149.65, 167.38, 169.09, 169.40; Anal Calc'd. for C₂₂H₂₄NO₇S: C, 57.38; H, 5.25; N, 6.08. Found: C, 57.31; H, 5.34; N, 5.83.

### 5.2. EXAMPLE 2: SYNTHESIS OF (+)2-[1-(3-ETHOXY-4-METHOXYPHENYL)-2-METHYLSULFONYLETHYL]-4-ACETYLAMINOISOINDOLINE-1,3-DIONE (for the purpose of reference)

### Preparation of 3-aminopthalic acid

10% Pd/C (2.5 g), 3-nitrophthalic acid (75.0 g, 355 mmol) and ethanol (1.5 L) were charged to a 2.5 L Parr hydrogenator under a nitrogen atmosphere. Hydrogen was charged to the reaction vessel for up to 55 psi. The mixture was shaken for 13 hours, maintaining hydrogen pressure between 50 and 55 psi. Hydrogen was released and the mixture was purged with nitrogen 3 times. The suspension was filtered through a celite bed and rinsed with methanol. The filtrate was concentrated *in vacuo.* The resulting solid was reslurried in ether and isolated by vacuum filtration. The solid was dried *in vacuo* to a constant weight, affording 54 g (84% yield) of 3-aminopthalic acid as a yellow product. ¹H-NMR (DMSO-d6) δ: 3.17 (s, 2H), 6.67 (d, 1H), 6.82 (d, 1H), 7.17 (t, 1H), 8-10 (br, s, 2H); ¹³C-NMR (DMSO-d6) δ: 112.00, 115.32, 118.20, 131.28, 135.86, 148.82, 169.15, 170.09.

### Preparation of 3-acetamidophthalic anhydride

A 1 L 3-necked round bottom flask was equipped with a mechanical stirrer, thermometer, and condenser and charged with 3-aminophthalic acid (108 g, 596 mmol) and acetic anhydride (550 mL). The reaction mixture was heated to reflux for 3 hours and cooled to about 25 °C and further to 0-5 °C for another 1 hour. The crystalline solid was collected by vacuum filtration and washed with ether. The solid product was dried *in vacuo* at ambient temperature to a constant weight, giving 75 g (61% yield) of 3-acetamidopthalic anhydride as a white product. ¹H-NMR (CDCl₃) δ: 2.21 (s, 3H), 7.76 (d, 1H), 7.94 (t, 1H), 8.42 (d, 1H), 9.84 (s, 1H).

### Resolution of 2-(3-ethoxy-4-methoxyphenyl-1-(methylsulphonyl)-eth-2-ylamine

A 3 L 3-necked round bottom flask was equipped with a mechanical stirrer, thermometer, and condenser and charged with 2-(3-ethoxy-4-methoxyphenyl)-1-(methylsulphonyl)-eth-2-ylamine (137.0 g, 500 mmol), N-acetyl-L-leucine (52 g, 300 mmol), and methanol (1.0 L). The stirred slurry was heated to reflux for 1 hour. The stirred mixture was allowed to cool to ambient temperature and stirring was continued for another 3 hours at ambient temperature. The slurry was filtered and washed with methanol (250 L). The solid was air-dried and then dried *in vacuo* at ambient temperature to a constant weight, giving 109.5 g (98% yield) of the crude product (85.8% ee). The crude solid (55.0 g) and methanol (440 mL) were brought to reflux for 1 hour, cooled to room temperature and stirred for an additional 3 hours at ambient temperature. The slurry was filtered and the filter cake was washed with methanol (200 mL). The solid was air-dried and then dried *in vacuo* at 30 °C to a constant weight, yielding 49.6 g (90% recovery) of (*S*)-2-(3-ethoxy-4-methoxyphenyl)-1-(methylsulphonyl)-eth-2-ylamine-N-acetyl-L-leucine salt (98.4% ee). Chiral HPLC (1/99 EtOH/20 mM KH₂PO₄ @ pH 7.0, Ultron Chiral ES-OVS from Agilent Technologies, 150 mm x 4.6 mm, 0.5 mL/min., @ 240 nm): 18.4 min (*S*-isomer, 99.2%), 25.5 min (*R*-isomer, 0.8%).

### Preparation of Compound A

A 500 mL 3-necked round bottom flask was equipped with a mechanical stirrer, thermometer, and condenser. The reaction vessel was charged with (*S*)-2-(3-ethoxy-4-methoxyphenyl)-1-(methylsulphonyl)-eth-2-yl amine N-acetyl-L-leucine salt (25 g, 56 mmol, 98% ee), 3-acetamidophthalic anhydride (12.1 g, 58.8 mmol), and glacial acetic acid (250 mL). The mixture was refluxed over night and then cooled to <50° C. The solvent was removed *in vacuo,* and the residue was dissolved in ethyl acetate. The resulting solution was washed with water (250 mL x 2), saturated aqueous NaHCO₃ (250 mL x 2), brine (250 mL x 2), and dried over sodium sulphate. The solvent was evaporated *in vacuo,* and the residue recrystallized from a binary solvent containing ethanol (150 mL) and acetone (75 mL). The solid was isolated by vacuum filtration and washed with ethanol (100 mL x 2). The product was dried *in vacuo* at 60 °C to a constant weight, affording 19.4 g (75% yield) of *S*-{2-[1-(3-ethoxy-4-methoxyphenyl)-2-methylsulfonylethyl]-4-acetamidoisoindoline-1,3-dione} with 98% ee. Chiral HPLC (15/85 EtOH/20 mM KH₂PO₄ @ pH 5, Ultron Chiral ES-OVS from Agilent Technology, 150 mm x 4.6 mm, 0.4 mL/min, @ 240 nm): 25.4 min (*S*-isomer, 98.7%), 29.5 min (R-isomer, 1.2%). ¹H-NMR (CDCl₃) δ: 1.47 (t, 3H), 2.26 (s, 3H), 2.87 (s, 3H), 3.68-3.75 (dd, 1H), 3.85 (s, 3H), 4.07-4.15 (q, 2H), 4.51-4.61 (dd, 1H), 5.84-5.90 (dd, 1H), 6.82-8.77 (m, 6H), 9.46 (s, 1H); ¹³C-NMR (DMSO-d6) δ: 14.66, 24.92, 41.61, 48.53, 54.46, 55.91, 64.51, 111.44, 112.40, 115.10, 118.20, 120.28, 124.94, 129.22, 131.02, 136.09, 137.60, 148.62, 149.74, 167.46, 169.14, 169.48.

Specific polymorphic solid forms of Compound A may be used in the dosage forms provided herein, as described in U.S. Patent Publication No. 2008/0234359.

### 5.3. EXAMPLE 3 (not in the scope of the claims): SYNTHESIS OF CYCLOPROPANECARBOXYLIC ACID {2-[(1S)-1-(3-ETHOXY-4-METHOXY-PHENYL)-2-METHANE-SULFONYL-ETHYL]-3-OXO-2,3-DIHYDRO-1H-ISOINDOL-4-YL}-AMIDE

### Preparation of methyl 2-methyl-6-nitrobenzoate

A mixture of 2-methyl-6-nitrobenzoic acid (300.0 g, 1.66 moles, from Acros Organics, Morris Plains, NJ) and trimethyl orthoacetate (298.3 g, 2.48 moles, from Aldrich Chemicals, Milwauke, WI) was charged into a 3-L 3-necked flask at about 20-25°C under nitrogen. The reaction mixture was gradually heated and the low-boiling point components generated during the reaction were distilled off to an internal temperature of 95-100°C. After 2 hours, the reaction mixture was cooled to 20-25°C over 1-2 hours. After heptane (1.50 L, from Aldrich Chemicals) was charged into the reaction mixture over 1.0-1.5 hours, the reaction mixture was seeded with methyl 2-methyl-6-nitrobenzoate (0.5 g) when it became turbid. The suspension was cooled to 0-5°C over 0.5-1 hour and kept at 0-5°C for another 1.5-2 hours. The solid was collected by filtration under vacuum, washed with heptane (3x300 mL), and dried to a constant weight in a tray at 30-35°C under a vacuum at 100-120 torr. The yield of methyl 2-methyl-6-nitrobenzoate was 292.0 g (91%), based on 300.0 g of 2-methyl-6-nitrobenzoic acid. The product was found to have a purity of >99% measured by HPLC based on area percentage, and a water content of <0.1% measured by Karl Fisher titration.

### Preparation of methyl 2-bromomethyl-6-nitrobenzoate

A mixture of methyl 2-methyl-6-nitrobenzoate (200.0 g, 1.02 moles, previously prepared), 1,3-dibromo-5,5-dimethylhydantoin (DBH, 162.0 g, 0.57 mole, from Aldrich Chemicals) and methyl acetate (1.20 L, from Aldrich Chemicals) was charged into a 3-L three-necked flask at about 20-25°C under nitrogen. After the reaction mixture was refluxed for 0.5-1 hour, a solution of 2,2'-azobisisobutyronitrile (AIBN, 8.6 g, 52 mmol, from Aldrich Chemicals) in 100 mL of methyl acetate was charged over 15-30 minutes. The reaction mixture was refluxed for 6.5-8 hours until the amount of unreacted 2-methyl-6-nitrobenzoate was less than 5-10%. The reaction mixture was cooled to 15-18°C and kept at 15-18°C for 50-60 minutes. The solid was filtered, washed with cold (i.e., 5-10°C) methyl acetate (2x100 mL) until there was less than 3% of methyl 2-bromomethyl-6-nitrobenzoate remained in the solid. Next, after heptane (1.00 L) was charged into the filtrate, the upper layer organic phase was washed with 2% of brine (2x500 mL) and deionized water (1-2 x 500 mL) until there was less than 0.5% (area percentage at 210 nm) of unreacted 5,5-dimethylhydantoin according to measurement by HPLC. After the solution was concentrated under a reduced pressure to remove about 1.80-1.90 L of methyl acetate, methyl tert-butyl ether (MTBE, 300 mL) was charged. After the reaction mixture was refluxed at 65-70°C for 10-15 minutes, the solution was cooled to 50-55°C over 0.5-1 hour and seeded with 500 mg of methyl 2-bromomethyl-6-nitrobenzoate at 45-50°C. The suspension was cooled to 20-25°C and kept at 20-25°C for 2-3 hours. The solids were collected by filtration, washed with 5-10°C a cold mixture of heptane and MTBE in a volume ratio of 1:2 (2x100 mL), and dried to a constant weight at 20-25°C under a vacuum at 100-120 torr. The yield of methyl 2-bromomethyl-6-nitrobenzoate was 185.2 g (66%), based on 200.0 g input of methyl 2-methyl-6-nitrobenzoate. The product was found to have a purity of >98% measured by HPLC based on area percentage, and a water content of <0.1% measured by Karl Fisher titration.

### Preparation of (1S)-1-(3-ethoxy-4-methoxyphenyl)-2-methanesulfonyl-ethylamine

After a mixture of (1S)-1-(3-ethoxy-4-methoxyphenyl)-2-methanesulfonyl-ethylamine N-acetyl-L-Leucine salt (1.10 kg, 2.46 moles), deionized water (4.40 L), and dichloromethane (DCM, 5.50 L) was charged into a reaction vessel, a solution of sodium hydroxide (196.0 g, 4.90 moles) in 1.00 L of deionized water was charged into the reaction vessel over about 5 minutes at 15-25°C. The resulting mixture was stirred for at least 10 minutes at 15-25°C and then the aqueous and organic phases were allowed to separate. The pH of the upper aqueous phase was maintained or adjusted at pH 13-14. The phases were separated and the upper aqueous phase was extracted with DCM (2x4.4 L). The pH of the aqueous phase was maintained at 13-14 throughout the extractions. The DCM extracts were combined and washed with deionized water (3.3 L) until the pH of the aqueous phase reached 11 or less. DCM was removed under vacuum below 35°C. The water content of the residual solid should be <0.1% w/w as measured by Karl Fisher titration. The residual solid was dried azeotropically with more DCM. The solid was dried to a constant weight in vacuo at 30-35°C to give (1S)-1-(3-ethoxy-4-methoxyphenyl)-2-methanesulfonyl-ethylamine as a white powder (639.0-672.0 g, 95-100% yield).

### Preparation of (1S)-7-nitro-2-[1-(3-ethoxy-4-methoxyphenyl)-2-(methylsulfonyl)ethyl]isoindolin-1-one

(1*S*)-7-nitro-2-[1-(3-ethoxy-4-methoxyphenyl)-2-(methylsulfonyl)ethyl]isoindolin-1-one was prepared by the following procedure. A mixture of methyl 2-bromomethyl-6-nitrobenzoate (100.0 g, 365 mmol, prepared previously in Example 5.7.2), (1S)-1-(3-ethoxy-4-methoxyphenyl)-2-methanesulfonylethylamine (104.7 g, 383 mmol, prepared previously in Example 5.7.3), sodium hydrogen carbonate (67.5 g, 8.03 moles, from Aldrich Chemicals) and dimethyl formamide (500 mL) was charged into a 1-L 3-necked flask at room temperature under nitrogen. The reaction mixture was gradually heated to an internal temperature of 70-75°C for two hours until there was less than <2% of unreacted methyl 2-bromomethyl-6-nitrobenzoate. The reaction mixture was gradually heated to an internal temperature of 95-100°C for 18 hours. The reaction mixture was cooled to 20-25°C and transferred to an 1-L addition funnel. After purified water (1500 mL) was charged into a 5-L 3-necked flask, the reaction mixture in the addition funnel was added into water in the 5-L 3-necked flask at room temperature over 1-2 hours maintaining an internal temperature below 30°C. The reaction mixture was stirred for 2 hours at room temperature. The solid was filtered out under vacuum, washed with water (3x300 mL) and methanol (2x400 mL), and then charged into a 2-L 3-necked flask followed by methanol (1000 mL). The mixture was refluxed for 1 hour. The mixture was cooled to room temperature. The solid was collected by filtration under vacuum, washed with 200 mL methanol (2 vol), and dried to a constant weight at 40-45°C under a vacuum at 100-120 torr. The yield of (1*S*)-7-nitro-2-[1-(3-ethoxy-4-methoxyphenyl)-2-(methylsulfonyl)ethyl]isoindolin-1-one was 123.0 g (78 %), based on 100.0 g input of methyl 2-bromomethyl-6-nitrobenzoate. The product was found to have a purity of >99% measured by HPLC based on area percentage, and a water content of <0.1% measured by Karl Fisher titration

### Alternative Preparation of (1S)-7-nitro-2-[1-(3-ethoxy-4-methoxyphenyl)-2-(methylsulfonyl)ethyl]isoindolin-1-one

(1*S*)-7-nitro-2-[1-(3-ethoxy-4-methoxyphenyl)-2-(methylsulfonyl)ethyl]isoindolin-1-one was also prepared by the following procedure. A mixture of methyl 2-bromomethyl-6-nitrobenzoate (100.0 g, 365 mmol, prepared previously in Example 5.7.2), (1S)-1-(3-ethoxy-4-methoxyphenyl)-2-methanesulfonyl-ethylamine (104.7 g, 383 mmol, prepared previously in Example 5.7.3), and potassium carbonate powder (100.8 g, 730 mmol, from Aldrich Chemicals) was suspended in acetonitrile (500 mL) at room temperature. The reaction mixture was refluxed at 81-83°C for about two hours until there was less than 2% of unreacted methyl 2-bromomethyl-6-nitrobenzoate. After the reaction mixture was cooled to 45-50°C, methanol (200 mL) was charged over 5-10 minutes. After the mixture was allowed to cool to 20-25°C and stirred for 2 hours, deionized water (1.40 L) was charged over 0.5-1 hour and stirred at 20-25°C for 30 minutes and at 0-5°C for 1-2 hours. The solid was filtered, washed with deionized water (3x300 mL), and dried to <10% of water content as measured by Karl Fisher titration. The solid was suspended in methanol (750 mL) and refluxed for 1-1.5 hours. The suspension was cooled to 0-5°C over 1.5-2 hours and kept at 0-5°C for 1-1.5 hours. The solid was filtered, washed with 0-5°C methanol (2x200 mL) and heptane (200 mL), and then dried at 40-45 °C under vacuum to a constant weight. The yield of (1S)-7-nitro-2-[1-(3-ethoxy-4-methoxyphenyl)-2-(methylsulfonyl)ethyl]isoindolin-1-one was 148.0 g (93 %), based on 100.0 g input of methyl 2-bromomethyl-6-nitrobenzoate. The product was found to have a purity of >99% measured by HPLC based on area percentage, and a water content of <1.0% measured by Karl Fisher titration

### Preparation of Compound B

A mixture of (1*S*)-7-nitro-2-[1-(3-ethoxy-4-methoxyphenyl)-2-(methylsulfonyl)ethyl]isoindolin-1-one (60 g, 138 mmol, prepared previously in Example 5.7.5), 10% Pd/C (50% wet, 2.4 g, 4 wt%, from Johnson Matthey, London, UK), ethyl acetate (780 mL) was charged into a Parr-vessel at room temperature under nitrogen. After the mixture was purged with nitrogen three times and with hydrogen three times, the reaction mixture was heated to 40°C and then the heat was removed. The reaction mixture was stirred with hydrogen at a pressure between 40-45 psi over 4-6 hours until there was ≤3% of the hydroxylamine intermediate. The reaction mixture was cooled to 20-25°C. The reaction mixture was filtered through a celite bed (1 inch thickness) and then bed-washed with ethyl acetate (120 mL). The filtrate was transferred to a 3-L 3-necked flask equipped with a 50-mL addition funnel. After N,N-diisopropylethylamine (29 mL, 165 mmol) was charged into the flask, the addition funnel was charged with cyclopropylcarbonyl chloride (13.0 mL, 145 mmol, from Aldrich Chemicals). The cyclopropylcarbonyl chloride was added at room temperature over 1-2 hours at an internal temperature below 30°C. The reaction mixture was stirred for 2-4 hours at room temperature. After heptane (300 mL) was added, the reaction mixture was stirred for 4-6 hours. The solid was collected by filtration under vacuum, washed with 2N HCl (2x300 mL), water (2x300 mL) and then heptane (2x300 mL). The crude product was dried at 40-45°C under a vacuum at 100-120 torr to a constant weight. The yield of crude Compound (1) was 58 g (88 %), based on 60.0 g input of (1*S*)-7-nitro-2-[1-(3-ethoxy-4-methoxyphenyl)-2-(methylsulfonyl)ethyl]-isoindolin-1-one.

### Recrystallization of Compound B

A mixture of crude Compound (1) (95.2 g, prepared previously in Example 5.7.6) and tetrahydrofuran (THF, 1.43 L) was charged into a 3L flask at 20-25°C under nitrogen. The suspension was heated to 60-65°C until dissolution was achieved. The suspension was filtered at 45-50°C and the solid was rinsed with 95 mL of THF prewarmed at 45-55°C. After about 950-1150 mL of THF was distilled off at normal pressure over 30-60 minutes, absolute ethanol (950 mL) was charged at 55-60°C over 5-10 minutes. About 350-400 mL of solvents was removed at normal pressure until the internal temperature rose to 72-74°C. The resulting suspension was refluxed at 72-75°C for 30-60 minutes, cooled to 20-25°C over 1-2 hours and kept at 20-25°C for another 1-2 hours. The solid was collected by filtration under vacuum, washed with absolute ethanol (240-280 mL) and heptane (240-280 mL), and then dried in tray at 50-55°C in a vacuum at 130-140 torr to a constant weight. The yield of the off-white crystalline product was (88.0-91.0 g, 92-96 %).

The compounds described herein may also be prepared according to the processes described in U.S. Patent Publication No. 2010/0168475.

### 5.4. EXAMPLE 4: CONTROLLED RELEASE FORMULATIONS 1 TO 3

Compound A was formulated in 500 mg tablets by direct compression. The drug loading is 10%. The data below show the *in vitro* evaluation of the release profile and water uptake of expandable polymer systems for gastroretentive system and controlled release solid dosage.

| **Formulation 1** | 500 mg | % | **Formulation 2** | 500 mg | % |
|---|---|---|---|---|---|
| Compound A | 50 | 10 | Compound A | 50 | 10 |
| POLYOX N-1105 | 180 | 36 | CMC 7L2P | 180 | 36 |
| NaCl | 75 | 15 | NaCl | 75 | 15 |
| Chitopharm S | 20 | 4 | ChitoClear 3568 | 20 | 4 |
| Protanal LF 200M | 75 | 15 | Protanal LF 200M | 75 | 15 |
| Lactose | 62.5 | 12.5 | Lactose | 62.5 | 12.5 |
| Ac-Di-Sol | 35 | 7 | Ac-Di-Sol | 35 | 7 |
| Mg Stearate | 2.5 | 0.5 | Mg Stearate | 2.5 | 0.5 |
| total | 500 | 100 | total | 500 | 100 |

| **Formulation 3** | 500 mg | % |
|---|---|---|
| Compound A | 50 | 10 |
| POLYOX 205 | 180 | 36 |
| NaCl | 75 | 15 |
| ChitoClear 3568 | 20 | 4 |
| Protanal LF 200M | 75 | 15 |
| Lactose | 62.5 | 12.5 |
| Ac-Di-Sol | 35 | 7 |
| Mg Stearate | 2.5 | 0.5 |
| total | 500 | 100 |

### Drug Release Profiles of Formulations 1 to 3

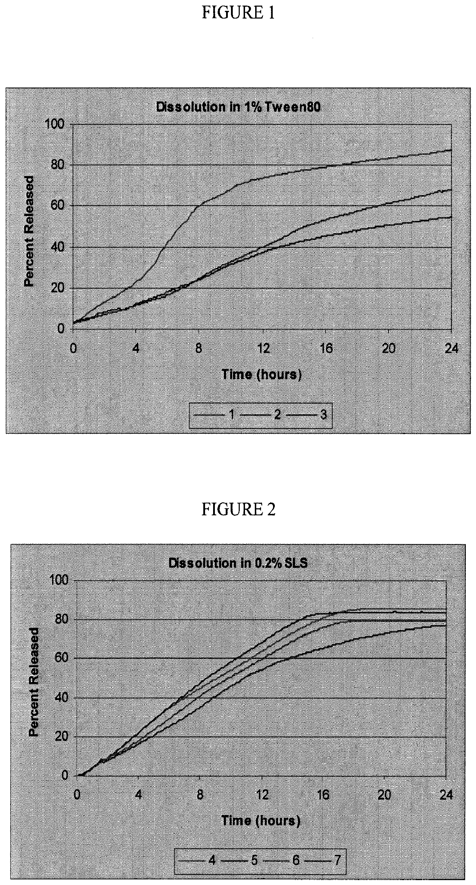

Drug dissolution studies from tablets were carried out in 900 mL dissolution medium, 1% Tween 80 solution with 10 mM NaAc at pH 4.0, at 100 RPM using USP I basket method. The drug content in tablets was 10%. Results are shown in FIG. 1.

### Swelling Profiles of Formulations 1 to 3

The swelling ratios of tablet Formulations 1 to 3 was determined by percent weight gain. Water uptake of the tablets was carried out in 500 mL solution at 37 °C with 10 mM NaAc at pH 4.0, using Distek Dissolution System.

| % Weight Gain | | | | |
|---|---|---|---|---|
| Formulation | 1 hr | 2 hr | 4 hr | 6 hr |
| 1 | 148 | 222 | 315 | 409 |
| 2 | 165 | 248 | 358 | 320 |
| 3 | 162 | 258 | 389 | 508 |

### 5.5. EXAMPLE 5: CONTROLLED RELEASE FORMULATIONS 4 TO 7

Compound A was formulated in 500 mg tablets by direct compression. The drug loading is 10%. The data below show the *in vitro* evaluation of the release profile and water uptake of expandable polymer systems for gastroretentive system and controlled release solid dosage. Formulations 4, 5, and 6 are not in the scope of the claims.

| **Formulation 4** | 500 mg | % | **Formulation 5** | 500 mg | % |
|---|---|---|---|---|---|
| Compound A | 50 | 10 | Compound A | 50 | 10 |
| POLYOX N-12K | 250 | 50 | POLYOX N-12K | 250 | 50 |
| Lactose | 57.5 | 11.5 | Lactose | 57.5 | 11.5 |
| Protanal LF 200M | 70 | 14 | Protanal LF 200D | 70 | 14 |
| Natrosol L Pharm | 70 | 14 | Natrosol L Pharm | 70 | 14 |
| Mg Stearate | 2.5 | 0.5 | Mg Stearate | 2.5 | 0.5 |
| total | 500 | 100 | total | 500 | 100 |

| **Formulation 6** | 500 mg | % | **Formulation 7** | 500 mg | % |
|---|---|---|---|---|---|
| Compound A | 50 | 10 | Compound A | 50 | 10 |
| POLYOX N-12K | 250 | 50 | POLYOX N-12K | 180 | 36 |
| Lactose | 57.5 | 11.5 | Lactose | 57.5 | 11.5 |
| Gelcarin GP 379 | 70 | 14 | Chitopharm S | 70 | 14 |
| | 70 | | Protanal LF | 70 | |
| Natrosol L Pharm | 2.5 | 14 | 200D | 70 | 14 |
| | | | Natrosol L | | |
| Mg Stearate | | 0.5 | Pharm | 2.5 | 14 |
| | | | Mg Stearate | | 0.5 |
| total | 500 | 100 | total | 500 | 100 |

### Drug Release Profiles of Formulations 4 to 7

Drug dissolution studies from tablets were carried out in 900 mL dissolution medium, 0.2% SLS with 10 mM NaAc at pH 4.0, at 100 RPM using USP I basket method. Results are shown in FIG. 2.

### Swelling Profiles of Formulations 4 to 7

The swelling ratios of tablet Formulations 4 to 7 was determined by percent weight gain. Water uptake of the tablets was carried out in 500 mL solution at 37 °C with 0.01 N HCl solution, using Distek Dissolution System.

| % Weight Gain | | | |
|---|---|---|---|
| Formulation | 1 hr | 2 hr | 6 hr |
| 4 | 132 | 176 | 249 |
| 5 | 147 | 180 | 249 |
| 6 | 139 | 176 | 209 |
| 7 | 156 | 252 | 664 |

### 5.6. EXAMPLE 6: CONTROLLED RELEASE FORMULATIONS 8 TO 11

Compound A was formulated in 250 mg tablets by direct compression. The drug loading is 20%. The data below show the *in vitro* evaluation of the release profile and water uptake of expandable polymer systems for gastroretentive system and controlled release solid dosage.

| **Formulation 8** | 250 mg | % | **Formulation 9** | 250 mg | % |
|---|---|---|---|---|---|
| Compound A | 50 | 20 | Compound A | 50 | 20 |
| POLYOX N-12K | 82 | 32.8 | POLYOX N-12K | 85 | 34 |
| NaCl powder | 26 | 10.4 | NaCl powder | 30 | 12 |
| Chitopharm M | 30 | 12 | Chitopharm S | 33 | 13.2 |
| Protanal LF 200M | 21 | 8.4 | Protanal LF 200M | 19 | 7.6 |
| Natrosol M Pharm | 21 | 8.4 | Natrosol G Pharm | 32 | 12.8 |
| Avicel PH-102 | 19 | 7.6 | Mg Stearate | 1 | 0.4 |
| Mg Stearate | 1 | 0.4 | | | |
| total | 250 | 100 | total | 250 | 100 |

| **Formulation 10** | 250 mg | % | **Formulation 11** | 250 mg | % |
|---|---|---|---|---|---|
| Compound A | 50 | 20 | Compound A | 50 | 20 |
| POLYOX N-12K | 85 | 34 | POLYOX N-12K | 85 | 34 |
| NaCl powder | 30 | 12 | NaCl powder | 15 | 6 |
| Eudragit E PO | 33 | 13.2 | Eudragit E PO | 33 | 13.2 |
| Protanal LF 200M | 19 | 7.6 | Lactose | 20 | 8 |
| Natrosol G Pharm | 32 | 12.8 | CMC 7LF | 19 | 7.6 |
| Mg Stearate | 1 | 0.4 | Natrosol G Pharm | 27 | 10.8 |
| | | | Mg Stearate | 1 | 0.4 |
| total | 250 | 100 | total | 250 | 100 |

### Drug Release Profiles of Formulations 8 to 11

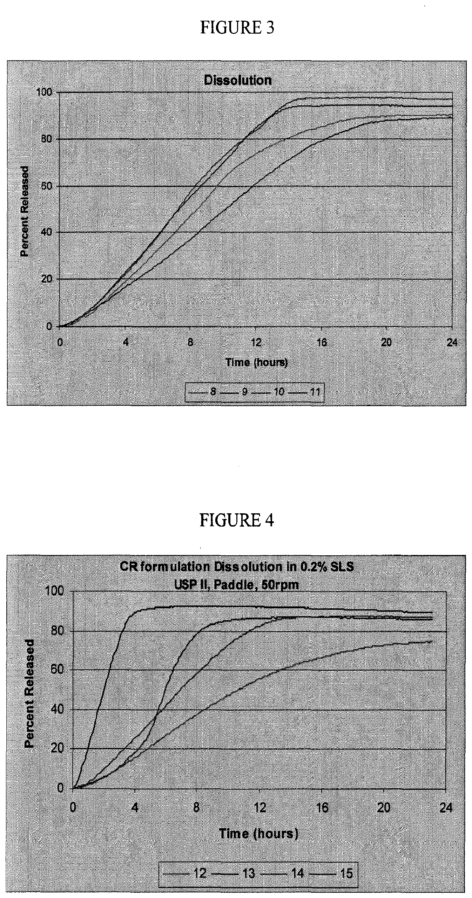

Drug dissolution studies from tablets were carried out in 900 mL dissolution medium, 0.2% SLS with 10 mM NaAc at pH 4.0, at 100 RPM using USP I basket method. Results are shown in FIG. 3.

### Swelling Profiles of Formulations 8 to 11

The swelling ratios of tablet Formulations 8 to 11 was determined by percent weight gain. Water uptake of the tablets was carried out in 500 mL solution at 37 °C with 0.01 N HCl solution, using Distek Dissolution System.

| % Weight Gain | | |
|---|---|---|
| Formulation | 1 hr | 2 hr |
| 8 | 197 | 252 |
| 9 | 175 | 219 |
| 10 | 175 | 164 |
| 11 | 186 | 187 |

### 5.7. EXAMPLE 7: CONTROLLED RELEASE FORMULATIONS 12 TO 15

The formulations of this example are not in the scope of the claims. Data shown below show the *in vitro* release profile of Formulations 12 to 15, which are controlled release tables without swelling effects. The compositions of Formulations 12 to 15 are in weight percent. The drug loading is 20%.

| **Formulation** | **12** | **13** | **14** | **15** |
|---|---|---|---|---|
| Compound A | 20 | 20 | 20 | 20 |
| HPMC E5LV | | 20 | | |
| HPMC K100LV | 30 | 10 | 30 | |
| Kollidon SR | | | | 10 |
| Polyox N-80 | | | | |
| Polyox 1105 | | | | |
| MCC | 49.5 | | | |
| Lactose | | 49.5 | 49.5 | 69.5 |
| Mg Stearate | 0.5 | 0.5 | 0.5 | 0.5 |

### Drug Release Profiles of Formulations 12 to 15

Drug dissolution studies from tablets were carried out in 900 mL dissolution medium, 0.2% SLS with 10 mM NaAc at pH 4.0, at 50 RPM using USP II paddle method. Results are shown in FIG. 4.

### 5.8. EXAMPLE 8: CONTROLLED RELEASE FORMULATIONS 16 TO 21

The formulations of this example are not in the scope of the claims. Data shown below show the *in vitro* release profile of Formulations 16 to 21, which are controlled release tables without swelling effects. The compositions of Formulations 16 to 21 are in weight percent. The drug loading is 20%.

| **Formulation** | **16** | **17** | **18** | **19** | **20** | **21** |
|---|---|---|---|---|---|---|
| Compound A | 20 | 20 | 20 | 20 | 10 | 10 |
| HPMC E5LV | 10 | | | | 20 | |
| HPMC K100LV | 20 | | | | 10 | 30 |
| Kollidon SR | | | | 20 | | |
| Polyox N-80 | | 20 | | | | |
| Polyox 1105 | | 10 | 30 | | | |
| MCC | | | | | | |
| Lactose | 49.5 | 49.5 | 49.5 | 59.5 | 59.5 | 59.5 |
| Mg Stearate | 0.5 | 0.5 | 0.5 | 0.5 | 0.5 | 0.5 |

### Drug Release Profiles of Formulations 16 to 21

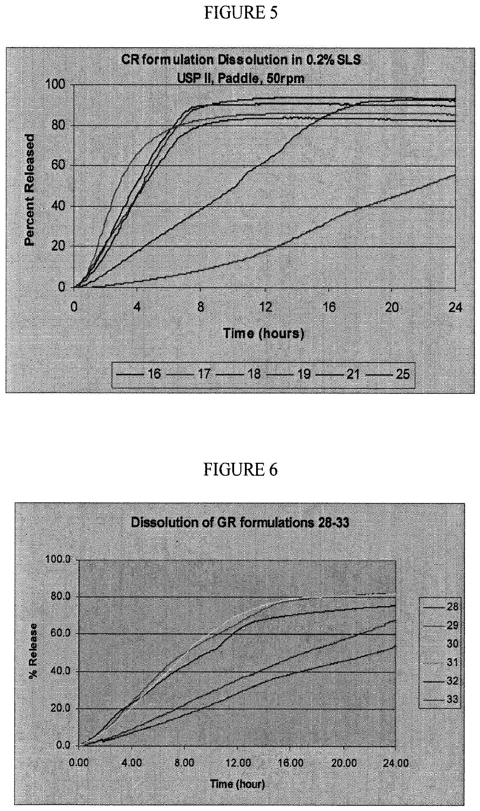

Drug dissolution studies from tablets were carried out in 900 mL dissolution medium, 0.2% SLS with 10 mM NaAc at pH 4.0, at 50 RPM using USP II paddle method. Results are shown in FIG. 5.

### 5.9. EXAMPLE 9: ADDITIONAL CONTROLLED RELEASE FORMULATIONS

The tables below present additional formulations of Compound A which were prepared and tested according to methods described herein for percent weight gain and percent drug release. Formulations 40, 41, 43, 44, 46-50, 55, and 90 are not in the scope of the claims.

| **Formulation** | **#28** | **#29** | **#30** | **#31** | **#32** | **#33** |
|---|---|---|---|---|---|---|
| Compound A | 20 | 20 | 20 | 20 | 20 | 20 |
| Polyox | 34 ^{a} | 34 ^{a} | 32.8 ^{a} | 30.4 ^{b} | 30.4 ^{b} | 34.4 ^{b} |
| NaCl Powder | 12 | 6 | 10.4 | 8.8 | 8.8 | |
| Chitopharm M | 13.2 | 13.2 | 12 | 13.2 | 13.2 | 13.2 |
| Protanal LF200M | 7.6 | | 8.4 | 7.6 | | 9.2 |
| Natrosol | 12.8 ^{c} | 10.8 ^{d} | 8.4 ^{d} | 12 ^{d} | 12 ^{d} | 9.2 ^{d} |
| Filler | | 8 ^{e} | 7.6 ^{f} | 7.6 ^{f} | 7.6 ^{f} | 13.6 ^{e} |
| CMC 7LF | | 7.6 | | | 7.6 | |
| Mg Stearate | 0.4 | 0.4 | 0.4 | 0.4 | 0.4 | 0.4 |
| a: Polyox N-12K | | | | | | |
| b: Polyox 301 | | | | | | |
| c: Natrosol L | | | | | | |
| d: Natrosol M | | | | | | |
| e: Lactose | | | | | | |
| f: Avicel | | | | | | |

| **Formulation** | **#34** | **#35** | **#36** | **#37** | **#38** | **#39** |
|---|---|---|---|---|---|---|
| Compound A | 20 | 20 | 20 | 20 | 20 | 20 |
| Polyox | 34 ^{a} | 33.2 ^{a} | 33.2 ^{a} | 33.2 ^{a} | 30.4 ^{b} | 34.4 ^{c} |
| NaCl Powder | 12 | 6 | 6 | 6 | 8.8 | |
| Chitopharm | 13.2 ^{d} | 13.2 ^{e} | 13.2 ^{e} | 13.2 ^{e} | 13.2 ^{e} | 13.2 ^{e} |
| Protanal LF200M | 7.6 | | 7.2 | | | 9.2 |
| Natrosol | 12.8 ^{f} | 8 ^{g} | 8 ^{g} | 8 ^{g} | 129 | 9.2 ^{g} |
| Filler | | 8 ^{h} | 8 ^{h} | 8 ^{h} | 7.6 ^{h} | 13.6 ^{h} |
| CMC 7LF | | 7.2 | | 7.2 | 7.6 | |
| Surfactant | | 4 ⁱ | 4 ^{j} | 4 ^{j} | | |
| Mg Stearate | 0.4 | 0.4 | 0.4 | 0.4 | 0.4 | 0.4 |
| a: Polyox N-12K | | | | | | |
| b: Polyox N-205 G | | | | | | |
| c: Polyox N-1105 | | | | | | |
| d: Chitopharm S | | | | | | |
| e: Chitopharm M | | | | | | |
| f: Natrosol G | | | | | | |
| g: Natrosol M | | | | | | |
| h: Lactose | | | | | | |
| i: SLS | | | | | | |
| j: Pluronic F108 | | | | | | |

| **Data** | **#28** | **#29** | **#30** | **#31** | **#32** | **#33** | **#34** | **#35** | **#36** | **#37** | **#38** | **#39** |
|---|---|---|---|---|---|---|---|---|---|---|---|---|
| % Weight gain (2 hr,) | 241 | 217 | 252 | 313 | 273 | 296 | 219 | 213 | 245 | 258 | 216 | 285 |
| % Release (8 hr) | 43 | 50 | 49 | 22 | 22 | 16 | 42 | 41 | 36 | 61 | 48 | 50 |
| % Release (24 hr) | 76 | 82 | 82 | 68 | 68 | 54 | 80 | 66 | 64 | 80 | 65 | 69 |

| **Formulation** | **#40** | **#41** | **#42** | **#43** | **#44** | **#45** |
|---|---|---|---|---|---|---|
| Compound A | 20 | 20 | 20 | 20 | 20 | 20 |
| Polyox N-K12 | 34 | 34 | 34 | 34 | 34 | 34 |
| NaCl Powder | 12 | 12 | 12 | 6 | 6 | 6 |
| Eudragit | 13.2 ^{a} | 13.2 ^{b} | 13.2 ^{c} | 13.2 ^{a} | 13.2 ^{b} | 13.2 ^{c} |
| Protanal LF200M | 7.6 | 7.6 | 7.6 | | | |
| Natrosol G | 12.8 | 12.8 | 12.8 | 10.8 | 10.8 | 10.8 |
| Lactose | | | | 8 | 8 | 8 |
| CMC 7LF | | | | 7.6 | 7.6 | 7.6 |
| Mg Stearate | 0.4 | 0.4 | 0.4 | 0.4 | 0.4 | 0.4 |
| a: Eudragit RLPO | | | | | | |
| b: Eudragit RSPO | | | | | | |
| c: Eudragit EPO | | | | | | |

| **Formulation** | **#46** | **#47** | **#48** | **#49** | **#50** | **#51** |
|---|---|---|---|---|---|---|
| Compound A | 20 | 20 | 20 | 20 | 20 | 20 |
| Polyox N-K12 | 50 | 50 | 50 | 50 | 50 | 36 |
| Chitopharm S | 14 | | | | | 14 |
| Protanal | | 14 ^{a} | 14 ^{b} | | | 14 ^{b} |
| Natrosol L | 14 | 14 | 14 | 14 | 14 | 14 |
| Gelcarin | | | | 14 ^{a} | 14 ^{d} | |
| Lactose | 11.5 | 11.5 | 11.5 | 11.5 | 11.5 | 11.5 |
| Mg Stearate | 0.5 | 0.5 | 0.5 | 0.5 | 0.5 | 0.5 |
| a: Protanal LF200D | | | | | | |
| b: Protanal LF200M | | | | | | |
| c: Gelcarin 379 | | | | | | |
| d: Gelcarin 209 | | | | | | |

| **Data** | **#40** | **#41** | **#42** | **#43** | **#44** | **#45** | **#46** | **#47** | **#48** | **#49** | **#50** | **#51** |
|---|---|---|---|---|---|---|---|---|---|---|---|---|
| % Weight gain (2 hr, HCl) | 175 | 173 | 164 | 181 | 188 | 187 | 147 | 176 | 180 | 176 | 175 | 252 |
| % Weight gain (6 hr, HCl) | 115 | 102 | 143 | 150 | 150 | 149 | 113 | 249 | 249 | 209 | 142 | 664 |
| % Release (8 hr) | 55 | 60 | 54 | 47 | 32 | 33 | 28 | 33 | 37 | 39 | 44 | 18 |
| % Release (24 hr) | 95 | 92 | 100 | 79 | 54 | 61 | 67 | 86 | 83 | 94 | 94 | 36 |

| **Formulation** | **#52** | **#53** | **#54** | **#55** | **#56** | **#57** |
|---|---|---|---|---|---|---|
| Compound A | 10 | 10 | 10 | 10 | 10 | 10 |
| Polyox N-10 | 13.5 | 13.5 | 13.5 | 13.5 | | 15.5 |
| Polyox N-1105 | 36 | 36 | 36 | 60 | 36 | 37 |
| Soluplus | | | | | 13.5 | |
| NaCl | 10 | 10 | 10 | 10 | 10 | 10 |
| Chitopharm S | 6 | 6 | 6 | | 6 | 3 |
| Protanal | 18 ^{a} | 18 ^{b} | 12 ^{b} | | 18 ^{b} | |
| Citric Acid | | | 6 | | | |
| Gelcarin | | | | | | 18 |
| Avicel PH102 | 6 | 6 | 6 | 6 | 6 | 6 |
| Mg Stearate | 0.5 | 0.5 | 0.5 | 0.5 | 0.5 | 0.5 |
| a: Protanal LF200 M | | | | | | |
| b: Protanal LF120 M | | | | | | |

| **Formulation** | **#58** | **#59** | **#60** | **#61** | **#62** | **#63** |
|---|---|---|---|---|---|---|
| Compound A | 10 | 10 | 10 | 10 | 10 | 10 |
| Polyox N-12K | 40 | 40 | 40 | 40 | 40 | 40 |
| NaCl | 15 | 15 | 15 | 15 | 15 | 15 |
| Chitopharm S | | | | | 10 | 10 |
| Chitoclear | 10 ^{a} | 10 ^{b} | 10 ^{c} | 10 ^{d} | | |
| Protanal LF200M | 16 | 16 | 16 | 16 | 16 | 6 |
| Gelcarin GP379 | | | | | | 10 |
| Avicel PH102 | 5.5 | 5.5 | 5.5 | 5.5 | 5.5 | 5.5 |
| Ac-Di-Sol | 3 | 3 | 3 | 3 | 3 | 3 |
| Mg Stearate | 0.5 | 0.5 | 0.5 | 0.5 | 0.5 | 0.5 |
| a: Chitoclear 3568 | | | | | | |
| b: Chitoclear 2832 | | | | | | |
| c: Chitoclear 3504 | | | | | | |
| d: Chitoclear 3548 | | | | | | |

| **Data** | **#52** | **#53** | **#54** | **#55** | **#56** | **#57** | **#58** | **#59** | **#60** | **#61** | **#62** | **#63** |
|---|---|---|---|---|---|---|---|---|---|---|---|---|
| % Weight gain (2 hr, pH 4) | 188 | 195 | 148 | 134 | 194 | 173 | 273 | 233 | 257 | 265 | 210 | 200 |
| % Weight gain (6 hr, pH 4) | 410 | 411 | 171 | 94 | 428 | 265 | 621 | 554 | 609 | 590 | 459 | 362 |
| % Release (8 hr) | 42 | 41 | 59 | 60 | 33 | 31 | 9 | 18 | 15 | 16 | 17 | 16 |
| % Release (24 hr) | 78 | 76 | 95 | 89 | 65 | 86 | 20 | 43 | 39 | 38 | 45 | 32 |

| **Formulation** | **#64** | **#65** | **#66** | **#67** | **#68** | **#69** |
|---|---|---|---|---|---|---|
| Compound A | 10 | 10 | 10 | 10 | 10 | 10 |
| Polyox N-1105 | 36 | | 36 | | | |
| NaCl | 15 | 15 | 15 | 15 | 15 | 15 |
| Chitopharm S | | | 4 | | | |
| Chitoclear 3568 | 4 | 4 | | 4 | 4 | 4 |
| Protanal | 15 | 15 | 15 | 15 | 15 | 15 |
| Swelling Agent | | 36 ^{a} | | 36 ^{b} | 36 ^{c} | 36 ^{d} |
| Lactose | 12.5 | 12.5 | 12.5 | 12.5 | 12.5 | 12.5 |
| Ac-Di-Sol | 7 | 7 | 7 | 7 | 7 | 7 |
| Mg Stearate | 0.5 | 0.5 | 0.5 | 0.5 | 0.5 | 0.5 |
| a: Natrosol M | | | | | | |
| b: CMC 7L2P | | | | | | |
| c: Polyox 205 | | | | | | |
| d: Natrosol G | | | | | | |

| **Formulation** | **#70** | **#71** | **#72** | **#73** | **#74** | **#75** |
|---|---|---|---|---|---|---|
| Compound A | 10 | 10 | 10 | 10 | 10 | 10 |
| Eudragit | | | | | 12 ^{a} | 12 ^{b} |
| Polyox N-1105 | 38 | 38 | | | | |
| Polyox N-12K | | | | | 38 | 38 |
| NaCl | 12 | 12 | 12 | 12 | 4 | 4 |
| Chitoclear 3568 | 4 | 4 | 4 | 4 | | |
| Protanal | 10 | 10 | 10 | 10 | | |
| Swelling Agent | | | 38 ^{c} | 38 ^{d} | 13.5 ^{e} | 13.5 ^{e} |
| Primojel | 10 | | | | | |
| Lactose | 15.5 | 16 | 15.5 | 15.5 | 12 | 12 |
| Ac-Di-Sol | | 10 | 10 | 10 | 10 | 10 |
| Mg Stearate | 0.5 | 0.5 | 0.5 | 0.5 | 0.5 | 0.5 |
| a: Eudragit EPO | | | | | | |
| b: Eudragit E100 | | | | | | |
| c: CMC 7LF | | | | | | |
| d: Natrosol G | | | | | | |
| e: CMC 7L2P | | | | | | |

| **Data** | **#64** | **#65** | **#66** | **#67** | **#68** | **#69** | **#70** | **#71** | **#72** | **#73** | **#74** | **#75** |
|---|---|---|---|---|---|---|---|---|---|---|---|---|
| % Weight gain (2 hr, pH 4) | 261 | 260 | 223 | 250 | 257 | 237 | 233 | 226 | 225 | 214 | 204 | 170 |
| % Weight gain (6 hr, pH 4) | 517 | 567 | 409 | 320 | 508 | 439 | 422 | 461 | 250 | 431 | 380 | 172 |
| % Release (8 hr) | 21 | 13 | 33 | 60 | 25 | 24 | 26 | 14 | 57 | 20 | 16 | 40 |
| % Release (24 hr) | 59 | 41 | 67 | 88 | 68 | 55 | 60 | 43 | 81 | 69 | 61 | 88 |

| **Formulation** | **#76** | **#77** | **#78** | **#79** | **#80** | **#81** |
|---|---|---|---|---|---|---|
| Compound A | 6.7 | 6.7 | 6.7 | 6.7 | 6.7 | 6.7 |
| Polyox N-1105 | 36 | 18 | | 39 | | 10 |
| NaCl | 15 | 15 | 15 | 15 | 14 | 14 |
| Chitoclear 3568 | 4 | 4 | 4 | 15 | 4 | |
| Protanal | 15 | 15 | 15 | 4 | 7.2 | |
| Swelling Agent | | 18 ^{a} | 36 ^{a} | | 42.8 ^{b} | 32 ^{a} |
| Eudragit EPO | | | | | | 10 |
| Lactose | 15.8 | 15.8 | 15.8 | 15.8 | 14.8 | 14.8 |
| Ac-Di-Sol | 7 | 7 | 7 | 7 | 10 | 12 |
| Mg Stearate | 0.5 | 0.5 | 0.5 | 0.5 | 0.5 | 0.5 |
| a: CMC 7L2P | | | | | | |
| b: CMC 7LF | | | | | | |

| **Formulation** | **#82** | **#83** | **#84** | **#85** | **#86** | **#87** |
|---|---|---|---|---|---|---|
| Compound A | 6.7 | 6.7 | 6.7 | 6.7 | 6.7 | 6.7 |
| Polyox N-1105 | | | 36 | 28 | | 25.1 |
| Polyox (other) | 33.6^{a} | | 14^{b} | 15.6^{b} | 10.7^{c} | |
| NaCl | 14 | 14 | 14 | 14 | 14.4 | 14 |
| Chitopharm S | 5.2 | 4 | 4 | 3.2 | 5.3 | 3.2 |
| Protanal | 12 ^{d} | 6.8^{d} | 12 ^{d} | | 17.1^{e} | |
| Swelling Agent | | 44^{f} | | 169 | | 14.59 |
| Lactose | 16 | 14 | 12.8 | 16 | 18.7 | 18 |
| MCC | | | | | 18.7 | 18 |
| Citric Acid | | | | | 8 | |
| Disintegrant | 12^{h} | 10ⁱ | | | | |
| Mg Stearate | 0.5 | 0.5 | 0.5 | 0.5 | 0.5 | 0.5 |
| a: Polyox N80 | | | | | | |
| b: Polyox N10 | | | | | | |
| c: Polyox N12K | | | | | | |
| d: Protanal LF200M | | | | | | |
| e: Protanal LF120M | | | | | | |
| f: CMC 7L2P | | | | | | |
| g: Gelcarin 209 | | | | | | |
| h: Primojel | | | | | | |
| i: Ac-Di-Sol | | | | | | |

| **Data** | **#76** | **#77** | **#78** | **#79** | **#80** | **#81** | **#82** | **#83** | **#84** | **#85** | **#86** | **#87** |
|---|---|---|---|---|---|---|---|---|---|---|---|---|
| % Weight gain (2 hr, pH 4) | 226 | 233 | 235 | 204 | 234 | 199 | 179 | 214 | 196 | 179 | 162 | 173 |
| % Weight gain (6 hr, pH 4) | 459 | 432 | 405 | 408 | 323 | 170 | 376 | 192 | 338 | 239 | 228 | 316 |
| % Release (8 hr) | 17 | 37 | 39 | 9 | 34 | 27 | 33 | 78 | 31 | 49 | 62 | 20 |
| % Release (24 hr) | 48 | 62 | 51 | 16 | 52 | 75 | 68 | 89 | 80 | 95 | 89 | 52 |

| **Formulation** | **#88** | **#89** | **#90** | **#91** | **#92** | **#93** |
|---|---|---|---|---|---|---|
| Compound A | 6.7 | 6.7 | 6.7 | 6.7 | 6.7 | 6.7 |
| Polyox N-750 | | | | | 20 | 10 |
| Polyox (other) | | | 14.4^{a} | 20^{b} | | 10^{c} |
| NaCl | 14 | 14 | 14 | 15 | 15 | 15 |
| Chitopharm S | 4 | | | | | |
| Chitoclear 3568 | | 4 | | 5 | 5 | 5 |
| Protanal LF200M | 6.8 | 12 | | 5 | 5 | 5 |
| Swelling Agent | 26.7^{d} | | 14.4^{e} | | | |
| Lactose | 14 | 14.4 | 16 | 47.8 | 47.8 | 47.8 |
| MCC | 16 | 14.4 | 16 | | | |
| Ac-Di-Sol | 10 | 10 | 10 | | | |
| Mg Stearate | 0.5 | 0.5 | 0.5 | 0.5 | 0.5 | 0.5 |
| a: Polyox N12K | | | | | | |
| b: Polyox N-1105 | | | | | | |
| c: Polyox N10 | | | | | | |
| d. CMC 7L2P | | | | | | |
| e: Gelcarin 379 | | | | | | |

| **Data** | **#88** | **#89** | **#90** | **#91** | **#92** | **#93** |
|---|---|---|---|---|---|---|
| % Weight gain (2 hr, pH 4) | 211 | 255 | 122 | 170 | -- | -- |
| % Weight gain (6 hr, pH 4) | 205 | 403 | 62 | 335 | -- | -- |
| % Release (8 hr) | 62 | 46 | 65 | 12 | 12 | 12 |
| % Release (24 hr) | 77 | 65 | 82 | 21 | 23 | 22 |

### 5.10. EXAMPLE 10: BILAYER TABLET FORMULATIONS

Bilayer tablets were prepared to achieve pulsatile drug release and/or immediate release followed by controlled release. An immediate release layer with a controlled release layer was been prepared for the bilayer tablets. The bilayer tablet was prepared as follows: load the gastric retentive portion (750mg) into the die and compress manually; load 100mg the immediate-release layer (Table 1) on top of it; compress using a Carver Press.

**Table 1: Formulation of Immediate-Released Layer**

| | |
|---|---|
| Compound A | 10.0 |
| Microcrystalline Cellulose | 26.25 |
| Lactose Monohydrate | 60.0 |
| Croscarmellose Sodium | 3.0 |
| Magnesium Stearate | 0.75 |
| Total | 100 |

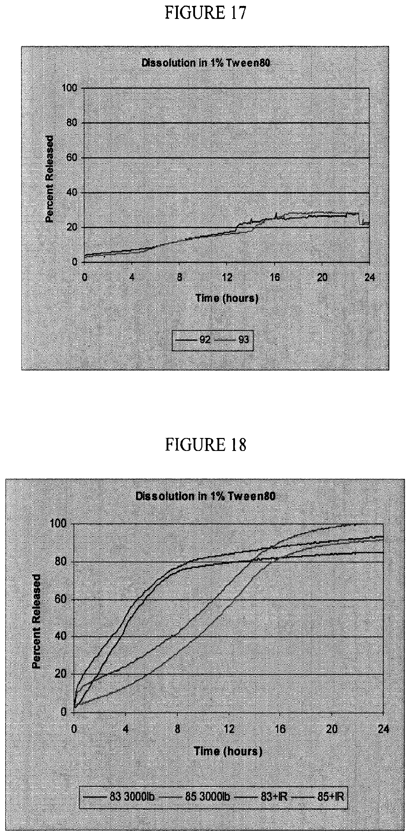

The release profiles of the bilayer tablets vs. 50 mg tablets at 3000 lb force are shown in Figure 18.

### 5.11. EXAMPLE 11: GASTRIC RETENTIVE BILAYER TABLET FORMULATIONS

Bilayer tablets were prepared to combine the gastric retentive function and extended release profile in one dose unit. Formulations 13, 14 and 16 were each used as the extended release layer. The formulation of the gastric retentive layer is provided as follows in Table 2. The bilayer tablet was prepared as follows: load the gastric retentive portion (500 mg) into the die and compress manually; load 250 mg the extended-release layer (*e.g*., Formulation 13, 14 or 16) on top of it; compress using a Carver Press.

**Table 2: Formulation of Gastric-Retentive Layer**

| | |
|---|---|
| Polyox 1105 | 16 |
| NaCl powder | 16 |
| Chitoclear 3568 | 12 |
| Protanal LF 200M | 12 |
| Avicel PH-102 | 43.5 |
| Mg Stearate | 0.5 |
| Total (%) | 100 |

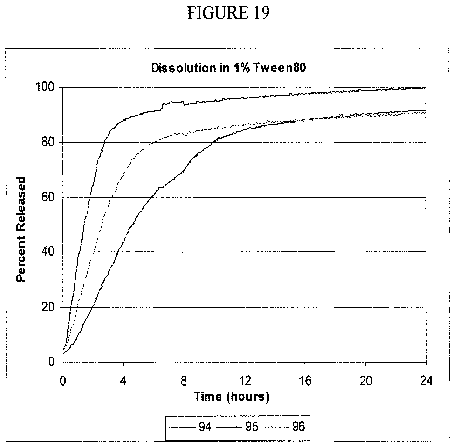

Bi-layer gastric-retentive tablets were prepared as Formulations 94, 95 and 96 (Table 3). The release profiles of the bi-layer tablets are show in Figure 19.

**Table 3: Formulation of Bi-layer Gastric-Retentive Tablets**

| Formulation # | ER Layer (250mg) | GR Layer (500mg) |
|---|---|---|
| 94 | 13 | 217 |
| 95 | 14 | 217 |
| 96 | 16 | 217 |

## Claims

1. A controlled release oral dosage form comprising:
(i) a compound of formula (I): 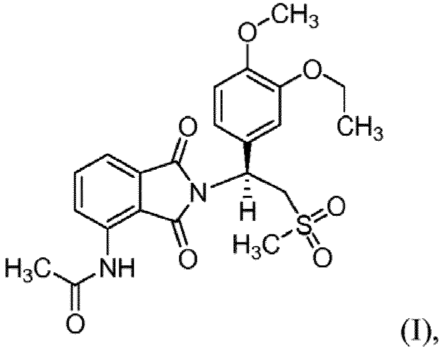 or a pharmaceutically acceptable polymorph, solvate or hydrate thereof;
(ii) a swelling excipient;
(iii) a cationic polymer in acidic pH;
(iv) an anionic polymer in acidic pH; and
wherein the swelling excipient is selected from the group consisting of hydroxyethylcellulose, polyethylene oxide, sodium carboxymethyl cellulose, hydroxypropyl cellulose, hydroxylpropyl methyl cellulose, methyl celluloses, croscarmellose sodium, sodium starch glycolate,
wherein the cationic polymer in acidic pH is selected from the group consisting of chitosan, methacrylic acidmethyl methacrylate copolymer (1:1), methacrylic acidmethyl methacrylate copolymer (1:2), poly(butyl methacylate-co-2-dimethylaminoethyl methacrylate-co-methyl methacrylate) (1:2:1), and crosslinked acrylic acid copolymers, and
wherein the anionic polymer in acidic pH is selected from the group consisting of an alginate, sodium carboxymethyl cellulose, chondroitin sulfate, carrageenan, glycosaminoglycans, mucopolysaccharides, pectin, gelatin and hyaluronic acid.

2. The controlled release oral dosage form of claim 1, wherein the cationic polymer in acidic pH is chitosan, the anionic polymer in acidic pH is an alginate and the swelling excipient is hydroxyethylcellulose or polyethylene oxide.

3. The controlled release oral dosage form of any of the preceding claims, wherein the anionic polymer in acidic pH is a sodium alginate.

4. The controlled release oral dosage form of any of the preceding claims, further comprising a disintegrant.

5. The controlled release oral dosage form of claim 4, wherein the disintegrant is selected from the group consisting of agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

6. The controlled release oral dosage form of claims 4 or 5, wherein the disintegrant is microcrystalline cellulose, croscarmellose sodium, or sodium starch glycolate.

7. The controlled release oral dosage form of any of the preceding claims, further comprising a lubricant.

8. The controlled release oral dosage form of claim 7, wherein the lubricant is selected from the group consisting of calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil, zinc stearate, ethyl oleate, ethyl laureate, agar, a syloid silica gel, a coagulated aerosol of synthetic silica, a pyrogenic silicon dioxide, and mixtures thereof.

9. The controlled release oral dosage form of any of the preceding claims, wherein the dosage form is a tablet or capsule.

10. The controlled release oral dosage form of claim 9, wherein the dosage form is a 5 mg, 10 mg, 15 mg, 20 mg, 25 mg, 30 mg, 40 mg, 50 mg, 60 mg, 75 mg, 100 mg, 150 mg, 200 mg, 250 mg, 300 mg, 400 mg, 500 mg, 750 mg or 1000 mg tablet.

## Patentansprüche

1. Orale Retard-Darreichungsform, umfassend:
(i) eine Verbindung der Formel (I): 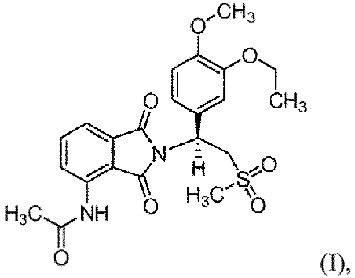 oder ein pharmazeutisch verträgliches Polymorph, Solvat oder Hydrat davon;
(ii) ein Quellmittel;
(iii) ein kationisches Polymer in saurem pH;
(iv) ein anionisches Polymer in saurem pH; und
wobei das Quellmittel aus der Gruppe bestehend aus Hydroxyethylcellulose, Polyethylenoxid, Natriumcarboxymethylcellulose, Hydroxypropylcellulose, Hydroxypropylmethylcellulose, Methylcellulosen, Croscarmellose-Natrium und Natriumstärkeglykolat ausgewählt ist,
wobei das kationische Polymer in saurem pH aus der Gruppe bestehend aus Chitosan, Methacrylsäure-Methylmethacrylat-Copolymer (1:1), Methacrylsäure-Methylmethacrylat-Copolymer (1:2), Poly(butylmethacrylat-co-2-dimethylaminoethylmethacrylat-co-methylmethacrylat) (1:2:1) und vernetzten Acrylsäurecopolymeren ausgewählt ist und
wobei das anionische Polymer in saurem pH aus der Gruppe bestehend aus einem Alginat, Natriumcarboxymethylcellulose, Chondroitinsulfat, Carrageenan, Glykosaminoglykanen, Mucopolysacchariden, Pektin, Gelatine und Hyaluronsäure ausgewählt ist

2. Orale Retard-Darreichungsform nach Anspruch 1, wobei das kationische Polymer in saurem pH Chitosan ist, das anionische Polymer in saurem pH ein Alginat ist und das Quellmittel Hydroxyethylcellulose oder Polyethylenoxid ist.

3. Orale Retard-Darreichungsform nach einem der vorhergehenden Ansprüche, wobei das anionische Polymer in saurem pH ein Natriumalginat ist.

4. Orale Retard-Darreichungsform nach einem der vorhergehenden Ansprüche, ferner umfassend ein Sprengmittel.

5. Orale Retard-Darreichungsform nach Anspruch 4, wobei das Sprengmittel aus der Gruppe bestehend aus Agar-Agar, Alginsäure, Calciumcarbonat, mikrokristalliner Cellulose, Croscarmellose-Natrium, Crospovidon, Polacrilin-Kalium, Natriumstärkeglykolat, Kartoffel- oder Tapiokastärke, vorgelatinierter Stärke, anderen Stärken, Tonen, anderen Alginen, anderen Cellulosen, Gummis und Gemischen davon ausgewählt ist.

6. Orale Retard-Darreichungsform nach Anspruch 4 oder 5, wobei das Sprengmittel mikrokristalline Cellulose, Croscarmellose-Natrium oder Natriumstärkeglykolat ist.

7. Orale Retard-Darreichungsform nach einem der vorhergehenden Ansprüche, ferner umfassend ein Gleitmittel.

8. Orale Retard-Darreichungsform nach Anspruch 7, wobei das Gleitmittel aus der Gruppe bestehend aus Calciumstearat, Magnesiumstearat, Mineralöl, leichtem Mineralöl, Glycerin, Sorbit, Mannit, Polyethylenglykol, anderen Glykolen, Stearinsäure, Natriumlaurylsulfat, Talkum, hydriertem Pflanzenöl, Zinkstearat, Ethyloleat, Ethyllaureat, Agar, einem Syloid-Kieselgel, einem koagulierten Aerosol von synthetischem Siliciumdioxid, einem pyrogenen Siliciumdioxid und Gemischen davon ausgewählt ist.

9. Orale Retard-Darreichungsform nach einem der vorhergehenden Ansprüche, wobei die Darreichungsform eine Tablette oder eine Kapsel ist.

10. Orale Retard-Darreichungsform nach Anspruch 9, wobei die Darreichungsform eine 5 mg, 10 mg, 15 mg, 20 mg, 25 mg, 30 mg, 40 mg, 50 mg, 60 mg, 75 mg, 100 mg, 150 mg, 200 mg, 250 mg, 300 mg, 400 mg, 500 mg 750 mg oder 1000 mg Tablette ist.

## Revendications

1. Forme posologique orale à libération contrôlée, comprenant
(i) un composé de formule (I) : 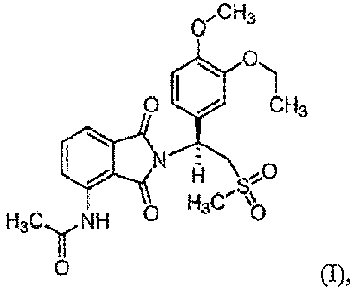 ou un polymorphe, un solvate ou un hydrate pharmaceutiquement acceptable de celui-ci ;
(ii) un excipient de gonflement ;
(iii) un polymère cationique en pH acide ;
(iv) un polymère anionique en pH acide ; et
l'excipient de gonflement étant choisi dans le groupe consistant en hydroxyéthylcellulose, poly(oxyde d'éthylène), carboxyméthylcellulose sodique, hydroxypropylcellulose, hydroxypropylméthylcellulose, méthylcelluloses, croscramellose sodique et glycolate d'amidon sodique,
le polymère cationique en pH acide étant choisi dans le groupe consistant en chitosane, copolymère d'acide méthacrylique-méthacrylate de méthyle (1/1), copolymère d'acide méthacrylique-méthacrylate de méthyle (1/2), poly(méthacrylate de butyle-co-méthacrylate de 2-diméthylaminoéthyle-co-méthacrylate de méthyle) (1/2/1) et copolymères d'acide acrylique réticulés, et
le polymère anionique en pH acide étant choisi dans le groupe consistant un alginate, carboxyméthylcellulose sodique, chondroïtine sulfate, carraghénine, glycosaminoglycanes, mucopolysaccharides, pectine, gélatine et acide hyaluronique.

2. Forme posologique orale à libération contrôlée selon la revendication 1, dans laquelle le polymère cationique en pH acide est le chitosane, le polymère anionique en pH acide est un alginate et l'excipient de gonflement est une hydroxyéthylcellulose ou un poly(oxyde d'éthylène).

3. Forme posologique orale à libération contrôlée selon l'une quelconque des revendications précédentes, dans laquelle le polymère anionique en pH acide est un alginate de sodium.

4. Forme posologique orale à libération contrôlée selon l'une quelconque des revendications précédentes, comprenant en outre un désintégrant.

5. Forme posologique orale à libération contrôlée selon la revendication 4, dans laquelle le désintégrant est choisi dans le groupe consistant en agar-agar, acide alginique, carbonate de calcium, cellulose microcristalline, croscarmellose sodique, crospovidone, polacriline-potassium, glycolate d'amidon sodique, amidon de pomme de terre ou de manioc, amidon prégélatinisé, autres amidons, argiles, autre algines, autres celluloses, gommes et mélanges de ceux-ci.

6. Forme posologique orale à libération contrôlée selon la revendication 4 ou la revendication 5, dans laquelle le désintégrant est une cellulose microcristalline, une croscarmellose sodique ou un glycolate d'amidon sodique.

7. Forme posologique orale à libération contrôlée selon l'une quelconque des revendications précédentes, comprenant en outre un lubrifiant.

8. Forme posologique orale à libération contrôlée selon la revendication 7, dans laquelle le lubrifiant est choisi dans le groupe consistant en stéarate de calcium, stéarate de magnésium, huile minérale, huile minérale légère, glycérine, sorbitol, mannitol, polyéthylène glycol, autres glycols, acide stéarique, laurylsulfate de sodium, talc, huile végétale hydrogénée, stéarate de zinc, oléate d'éthyle, lauréate d'éthyle, agar, un gel de silice Syloid, un aérosol coagulé de silice synthétique, un dioxyde de silicium pyrogène et des mélanges de ceux-ci.

9. Forme posologique orale à libération contrôlée selon l'une quelconque des revendications précédentes, dans laquelle la forme posologique est un comprimé, un comprimé en forme de capsule, une capsule ou un liquide.

10. Forme posologique orale à libération contrôlée selon la revendication 9, la forme posologique étant un comprimé de 5 mg, 10 mg, 15 mg, 20 mg, 25 mg, 30 mg, 40 mg, 50 mg, 60 mg, 75 mg, 100 mg, 150 mg, 200 mg, 250 mg, 300 mg, 400 mg, 500 mg, 750 mg ou 1000 mg.
